# EUROPÄISCHE PATENTSCHRIFT

(11) **EP 3 806 415 B1**
(45) Veröffentlichungstag und Bekanntmachung des Hinweises auf die Patenterteilung: **14.06.2023**
(21) Anmeldenummer: 19202043.6
(22) Anmeldetag: 08.10.2019
(51) Int. Cl.: H04L 9/40, G06F 21/60, G06Q 10/06

(54) **VERFAHREN ZUM SICHEREN AUSFÜHREN EINES WORKFLOWS IN EINEM COMPUTERSYSTEM**
METHOD FOR SECURE EXECUTION OF A WORKFLOW IN A COMPUTER SYSTEM
PROCÉDÉ D'EXÉCUTION SÉCURISÉE D'UN FLUX DE TRAVAIL DANS UN SYSTÈME INFORMATIQUE

(43) Veröffentlichungstag der Anmeldung: 14.04.2021
(73) Patentinhaber: Camelot ITLAB GmbH, 68165 Mannheim (DE)
(72) Erfinder: Göbel, Andreas, 76694 Forst (DE); Joswig, Steffen, 69514 Laudenbach (DE); Packowski, Josef, 69121 Heidelberg (DE)
(74) Vertreter: 2s-ip Schramm Schneider Bertagnoll Patent- und Rechtsanwälte Part mbB

(56) Entgegenhaltungen:
- US-A1- 2009 112 873
- US-B1- 7 805 327
- ORLENYS L\'OPEZ-PINTADO ET AL: "CATERPILLAR: A Business Process Execution Engine on the Ethereum Blockchain", ARXIV.ORG, CORNELL UNIVERSITY LIBRARY, 201 OLIN LIBRARY CORNELL UNIVERSITY ITHACA, NY 14853, 10. Juli 2018 (2018-07-10), XP081254625,
- JOERG EVERMANN ET AL: "Workflow Management on BFT Blockchains", ARXIV.ORG, CORNELL UNIVERSITY LIBRARY, 201 OLIN LIBRARY CORNELL UNIVERSITY ITHACA, NY 14853, 29. Mai 2019 (2019-05-29), XP081365632,

## Beschreibung

### Gebiet der Erfindung

Die Erfindung betrifft ein Computer-implementiertes Verfahren zum sicheren Ausführen eines Workflows in einem Computersystem.

### Hintergrund der Erfindung

Es ist bekannt, Workflows in einem verteilten Netzwerk auszuführen, wobei mehrere Teilnehmer an der Ausführung eines Workflows beteiligt sind. Jeder dieser Teilnehmer kann andere Schritte des Workflows ausführen, wobei der Workflow-Schritt auf einem anderen Clientsystem in dem verteilten Netzwerk ausgeführt werden kann. Die Veröffentlichungen US 2009/112873 und ""CATERPILLAR: A Business Process Execution Engine on the Ethereum Blockchain" offenbaren Verfahren zum sicheren Ausführen eines Workflows Solche Workflowsysteme haben allerdings den Nachteil, dass einerseits nicht gewährleistet werden kann, dass die Definition eines Workflows, also das Workflow-Modell, nicht manipuliert wird. Andererseits kann auch nicht gewährleistet werden, dass der ausgeführte Workflow, also die Workflow-Instanz eines Workflow-Modells, nicht manipuliert wird. Beide Arten der Manipulation können dazu führen, dass ein Workflow nicht so ausgeführt wird, wie von den Teilnehmern erwartet. Beispielsweise kann eine manipulierte Workflow-Instanz dazu führen, dass
- vertrauenswürdige Daten preisgegeben werden, oder
- Workflow-Schritte ausgeführt werden, die nicht ausgeführt werden dürften, oder
- Daten in den Computer-Systemen manipuliert werden.

### Aufgabe der Erfindung

Aufgabe der vorliegenden Erfindung ist es daher, eine Lösung bereitzustellen, die ein sicheres Ausführen einer Workflow-Instanz in einem Computersystem ermöglicht, wobei sowohl die Workflow-Instanz als auch das der Workflow-Instanz zugrunde liegende Workflow-Modell vor Manipulationen geschützt ist.

### Erfindungsgemäße Lösung

Bereit gestellt wird demnach ein Computer-implementiertes Verfahren zum sicheren Ausführen eines Workflows in einem Computersystem, das zumindest ein verteiltes Repository-Netzwerk und eine Anzahl von mit diesem Repository-Netzwerk operativ gekoppelte oder operativ koppelbare Clientsysteme umfasst, wobei
- in dem Repository-Netzwerk ein Modell des Workflows gespeichert wird,
- das Modell des Workflows eine Anzahl von Workflow-Schritten umfasst, wobei ein vorbestimmter Workflow-Schritt als Initialschritt, der beim Ausführen des Workflows in dem Computersystem als erster Schritt ausgeführt wird, definiert ist, und wobei für jeden Workflow-Schritt in dem Modell des Workflows gespeichert sind
   - Ausführungsbedingungen, die zum Ausführen des Workflow-Schritts in dem Computersystem erfüllt sein müssen,
   - Ereignisse (d.h. Informationen über Ereignisse), die beim Ausführen des Workflow-Schrittes in dem Computersystem aufgezeichnet werden,
   - Aufgaben (d.h. Information über Aufgaben), die einem Benutzer, einer Benutzerrolle oder einem Clientsystem zugeordnet werden, wenn ein Workflow-Schritt in dem Computersystem ausgeführt wird, und
   - Benachrichtigungen (d.h. Informationen über Benachrichtigungen), die einem Benutzer, einer Benutzerrolle oder einem Clientsystem zur Verfügung gestellt werden, wenn ein Workflow-Schritt in dem Computersystem erfolgreich ausgeführt wurde oder wenn ein Workflow-Schritt in dem Computersystem ausgeführt werden muss, und Informationen zu einer Anzahl von Aktionen,
- zum Ausführen des Workflows in dem Computersystem aus dem Modell des Workflows eine Workflow-Instanz erzeugt wird, die den auszuführenden Workflow repräsentiert, wobei die Workflow-Instanz in dem Repository-Netzwerk gespeichert wird,
- beim Ausführen der Workflow-Instanz in dem Computersystem
   - zunächst der Initialschritt und anschließend die weiteren Workflow-Schritte ausgeführt werden, wobei beim Ausführen eines Workflow-Schrittes die Aktionen ausgeführt werden,
   - beim Starten eines Workflow-Schritts die Ereignisse, die Benachrichtigungen und basierend auf den Informationen zu der Anzahl von Aktionen die Aktionen erzeugt (d.h. gemäß der jeweiligen in dem Workflow-Modell gespeicherten Informationen) und in dem Repository-Netzwerk gespeichert werden,
   - für jeden ausgeführten Workflow-Schritt Zustandsdaten in dem Repository-Netzwerk gespeichert werden, und
   - eine auf zumindest einem Clientsystem ausgeführte Workflow-Applikation
      - Benachrichtigungen von dem Repository-Netzwerk empfängt, die für einen vorbestimmten Benutzer in dem Repository-Netzwerk gespeichert wurden, und
      - für einen auszuführenden Workflow-Schritt auf dem zumindest einen Clientsystem einen dem Workflow-Schritt zugeordneten Programmcode ausführt.

Vorteilhaft ist es, wenn das verteilte Repository-Netzwerk ein Blockchain-Netzwerk ist.

Dadurch ist gewährleistet, dass das Modell des Workflows unveränderlich gespeichert ist. Zudem ist auch die Workflow-Instanz unveränderlich gespeichert. Manipulationen des Workflow-Modells und der Workflow-Instanzen können so effektiv verhindert werden. Durch das Speichern der Zustandsdaten in dem Repository-Netzwerk kann zudem eine unveränderliche Protokollierung der Ausführung einer Workflow-Instanz erreicht werden.

In dem Modell des Workflows können für einen Workflow-Schritt Informationen zu einer Anzahl von Aktionen gespeichert sein, wobei die Aktionen beim Ausführen des Workflow-Schrittes ausgeführt werden, und wobei beim Starten des Workflow-Schrittes basierend auf den Informationen zu der Anzahl von Aktionen die Aktionen erzeugt werden und in dem Repository-Netzwerk gespeichert werden.

Vorteilhaft ist es, wenn der einem Workflow-Schritt zugeordnete Programmcode als Bestandteil der Workflow-Applikation auf dem Clientsystem gespeichert wird.

Besonders vorteilhaft ist es aber, wenn der einem Workflow-Schritt zugeordnete Programmcode in dem Repository-Netzwerk gespeichert wird. Dadurch ist gewährleistet, dass auch der Programmcode unveränderlich gespeichert wird.

Die Zustandsdaten können einen Zeitstempel, der den Ausführungszeitpunkt des Workflow-Schrittes angibt, eine Benutzerkennung des Benutzers, der den Workflow-Schritt ausgeführt hat, und einen Status des Workflow-Schrittes umfassen.

Beim Ausführen des dem Workflow-Schritt zugeordneten Programmcodes durch die Workflow-Applikation können der Workflow-Applikation Daten übergeben werden, die zur Ausführung des Programmcodes notwendig sind.

Die übergebenen Daten können in einer Speichereinrichtung des Clientsystems, auf dem der Programmcode ausgeführt wird, und/oder in dem verteilten Repository-Netzwerk gespeichert sein.

Beim Ausführen des dem Workflow-Schritt zugeordneten Programmcodes durch die Workflow-Applikation können Daten erzeugt werden. Die erzeugten Daten können in einer Speichereinrichtung des Clientsystems, auf dem der Programmcode ausgeführt wird, und/oder in dem verteilten Repository-Netzwerk gespeichert werden.

Vorteilhaft ist es, wenn die erzeugten Daten verschlüsselt in dem Repository-Netzwerk gespeichert werden.

In einer Ausgestaltung der Erfindung kann das Modell des Workflows einen ersten und zumindest einen zweiten Teilworkflow umfassen, wobei jeder Teilworkflow eine Anzahl von Workflow-Schritte umfasst, und wobei ein Modell des ersten Teilworkflows in einem ersten Clientsystem der Anzahl von Clientsystemen und ein Modell des zumindest einen zweiten Teilworkflows in einem zweiten Clientsystem der Anzahl von Clientsystemen erzeugt wird, wobei die Modelle der Teilworkflows kombiniert werden und als Modell des Workflows in dem verteilten Repository-Netzwerk gespeichert wird.

Jedem Modell eines Teilworkflows kann ein Benutzer und/oder eine Benutzerrolle zugeordnet werden, wobei mit dieser Zuordnung dem Benutzer und/oder der Benutzerrolle Rechte zum Verwalten, insbesondere Rechte zum Ändern des jeweiligen Modells des Teilworkflows, eingeräumt werden.

Vorteilhaft ist es, wenn vor dem Ausführen eines Workflow-Schritts von der Workflow-Applikation geprüft wird, ob die Ausführungsbedingungen zum Ausführen des Workflow-Schritts erfüllt sind.

Die Ausführungsbedingungen umfassen zumindest eines aus der Gruppe
- der auszuführende Workflow-Schritt muss ein gültiger Workflow-Schritt sein, wobei ein auszuführender Workflow-Schritt dann gültig ist, wenn vorangegangene Workflow-Schritte, von denen der auszuführende Workflow-Schritt abhängt, erfolgreich ausgeführt wurden,
- der Benutzer, der den auszuführenden Workflow-Schritt startet, muss eine Berechtigung zum Ausführen des Workflow-Schritts haben, wobei die Berechtigungen in dem verteilten Repository-Netzwerk gespeichert werden, und
- Kombination hiervon.

### Kurzbeschreibung der Figuren

Einzelheiten und Merkmale der Erfindung ergeben sich aus der nachfolgenden Beschreibung in Verbindung mit der Zeichnung. Es zeigt:
- Fig. 1: ein Blockdiagramm eines Computersystems zum sicheren Ausführen eines Workflows,
- Fig. 2: ein Blockdiagramm, anhand dessen das Verfahren zum sicheren Ausführen eines Workflows näher beschrieben wird;
- Fig. 3 und 4: ein Beispiel eines Workflow-Modells;
- Fig. 5: einen exemplarischen Ablauf für das Erzeugen eines Trustlets;
- Fig. 6: ein Blockdiagramm, anhand dessen das Verfahren zum sicheren Validieren von vertrauenswürdigen Daten näher beschrieben wird; und
- Fig. 7: ein Ablaufdiagramm zur Beschreibung eines konkreten Beispiels des Verfahrens zum sicheren Validieren von vertrauenswürdigen Daten.

### Detaillierte Beschreibung der Erfindung

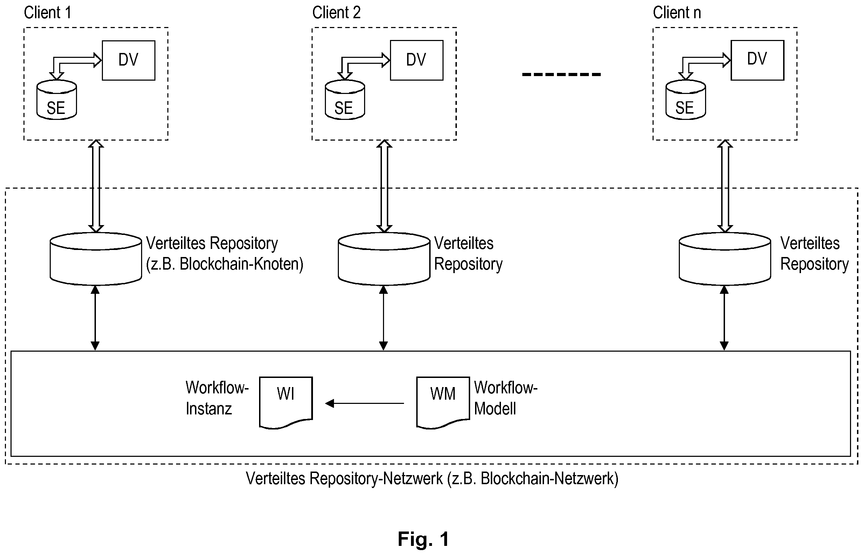

**Fig. 1** zeigt ein Blockdiagramm eines Computersystems zum sicheren Ausführen eines Workflows. Das Computersystem umfasst ein verteiltes Repository-Netzwerk, das vorzugsweise als Blockchain-Netzwerk, bestehend aus mehreren verteilten Blockchain-Knoten, ausgestaltet ist. Ferner umfasst das Computersystem eine Anzahl von Clientsystemen (Client 1, Client 2, ..., Client n), die operativ mit dem verteilten Repository-Netzwerk gekoppelt sind bzw. koppelbar sind. Vorzugsweise umfassen die Clientsysteme jeweils ein Datenverarbeitungssystem DV, die ihrerseits jeweils zumindest eine Speichereinrichtung SE (lokale Speichereinrichtung) umfassen.

Die Blockchain-Knoten können jeweils den einzelnen Clientsystemen zugeordnet sein, d.h., die Daten dieser Blockchain-Knoten können jeweils in einer Speichereinrichtung der Clientsysteme gespeichert sein. Alternativ können die Blockchain-Knoten aber auch entfernt von den jeweiligen Clientsystemen gehalten werden, wobei die Clientsysteme über ein Kommunikationsnetzwerk (etwa das Internet) auf die Blockchain-Knoten zugreifen können.

An einem der Clientsysteme wird ein Modell eines Workflows (nachfolgend auch Workflow-Modell genannt) erzeugt und in dem verteilten Repository-Netzwerk gespeichert. Das Workflow-Modell steht dann zur Ausführung durch die Clientsysteme zur Verfügung.

Zum Ausführen des durch das Workflow-Modell definierten Workflows wird in dem Computersystem aus dem Workflow-Modell eine Workflow-Instanz erzeugt. Diese Workflow-Instanz wird dann wiederum in dem verteilten Repository-Netzwerk gespeichert. Ausführungsbedingungen und der genaue Ablauf beim Ausführen eines Workflows bzw. einer Workflow-Instanz werden weiter unten näher beschrieben.

Beim Ausführen eines Workflows, d.h. einer Workflow-Instanz können Daten erzeugt werden, die wiederum in dem verteilten Repository-Netzwerk gespeichert werden können. Alternativ oder zusätzlich können die beim Ausführen einer Workflow-Instanz erzeugten Daten auch in den lokalen Speichereinrichtungen SE der Clientsysteme gespeichert werden.

Ferner können zum Ausführen einer Workflow-Instanz auch Daten zur Verfügung gestellt werden, die ebenfalls in dem verteilten Repository-Netzwerk und/oder in den lokalen Speichereinrichtungen SE der Clientsysteme gespeichert sein können.

Auf den Clientsystemen, die an der Ausführung einer Workflow-Instanz beteiligt sind, werden sogenannte Workflow-Applikationen ausgeführt. Für jeden auszuführenden Workflow-Schritt wird von der Workflow-Applikation ein vorbestimmter Programmcode zur Ausführung gebracht. Bei dem Programmcode kann es sich um einen sogenannten Chaincode oder Smart Contract handeln.

Der Programmcode für einen Workflow-Schritt kann in dem jeweiligen Clientsystem, etwa als Bestandteil der jeweiligen Workflow-Applikation gespeichert sein. Alternativ kann der Programmcode auch in dem verteilten Repository-Netzwerk gespeichert werden und zur Ausführung an das jeweilige Clientsystem bzw. an die jeweilige Workflow-Applikation übertragen werden. In einer Ausgestaltung der Erfindung kann der Programmcode auch auf einem Blockchain-Knoten zur Ausführung gebracht werden.

Ein Workflow-Schritt kann mehrere Aktionen umfassen. In diesem Fall kann der Programmcode des Workflow-Schrittes mehrere Programmcode-Abschnitte umfassen, wobei jeder Aktion des Workflow-Schrittes ein Programmcode-Abschnitt zugeordnet ist.

Eine Workflow-Instanz kann mehrere Workflow-Schritte umfassen, wobei jeder Workflow-Schritt auf einem anderen Clientsystem bzw. von einer anderen Workflow-Applikation ausgeführt werden kann.

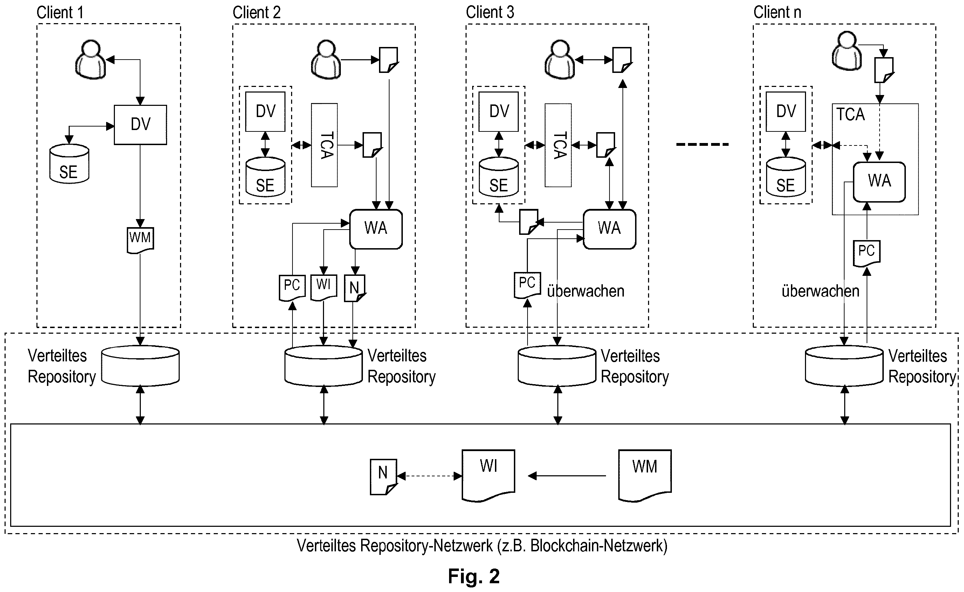

**Fig. 2** zeigt ein Blockdiagramm, anhand dessen das Verfahren zum sicheren Ausführen eines Workflows näher beschrieben wird.

Bei dem in Fig. 2 gezeigten Beispiel sind vier Personen an der Modellierung bzw. an der Ausführung eines Workflows beteiligt, wobei jede Person einem Clientsystem zugeordnet ist.

Das gesamte Verfahren lässt sich prinzipiell in 3 Hauptschritte unterteilen:

| | |
|---|---|
| - Hauptschritt 1: | Erzeugen des Workflow-Modells; |
| - Hauptschritt 2: | Erzeugen einer Workflow-Instanz, d.h. Starten eines Workflows basierend auf einem Workflow-Modell; und |
| - Hauptschritt 3: | Ausführen der Workflow-Instanz. |

### Hauptschritt 1: Erzeugen des Workflow-Modells

Der Hauptschritt 1, d.h. das Erzeugen eines Workflow-Modells, wird bei dem in Fig. 2 gezeigten Beispiel an dem ersten Clientsystem (Client 1) ausgeführt. Hierzu kann auf der Datenverarbeitungseinrichtung DV des ersten Clientsystems eine Software zur Modellierung von Workflows ausgeführt werden, mit der das gewünschte Workflow-Modell erstellt werden kann.

Ein Prozess, der mit einem Workflow-Modell beschrieben wird, besteht in der Regel aus mehreren Prozessschritten, wobei an dem Prozess mehrere Teilnehmer beteiligt sein können.

Ein Workflow-Modell beschreibt eine Anzahl von Workflow-Schritten, wobei ein Prozessschritt in dem Workflow-Modell durch mehrere Workflow-Schritte repräsentiert werden kann. Im einfachsten Fall wird ein Prozessschritt durch einen Workflow-Schritt repräsentiert. Zudem beschreibt ein Workflow-Modell Interaktionen mit Benutzern (Teilnehmer, die an dem Prozess beteiligt sind) und welche Daten für die Ausführung des Workflows benötigt werden und welche Daten beim Ausführen des Workflows erzeugt werden.

Das erzeugte Workflow-Modell wird in dem verteilten Repository-Netzwerk, vorzugsweise ein Blockchain-Netzwerk, gespeichert.

Erfindungsgemäß wird in dem Workflow-Modell für jeden Workflow-Schritt zumindest Folgendes gespeichert bzw. definiert:
- Ausführungsbedingungen, die zum Ausführen des jeweiligen Workflow-Schritts in dem Computersystem erfüllt sein müssen. Das können beispielsweise Berechtigungen sein, über die ein Benutzer verfügen muss, um den Workflow-Schritt auszuführen. Eine weitere Ausführungsbedingung könnte beispielsweise definieren, welche vorhergehenden Workflow-Schritte erfolgreich abgeschlossen sein müssen, um den Workflow-Schritt ausführen zu können. Eine noch weitere Ausführungsbedingung könnte beispielsweise definieren, welche Daten in dem verteilten Repository-Netzwerk und/oder in der lokalen Speichereinrichtungen des Clientsystems, auf dem der Workflow-Schritt ausgeführt werden soll, vorhanden sein müssen, um den Workflow-Schritt ausführen zu können.

Die Ausführungsbedingungen für die Ausführung des Workflows bzw. der Workflow-Instanz an sich sind durch die Ausführungsbedingungen des Initialschrittes (Workflow-Schritt, der als erster Schritt beim Starten der Workflow-Instanz ausgeführt wird) der Workflow-Instanz festgelegt.
- Ereignisse, die beim Ausführen des Workflow-Schrittes in dem Computersystem aufgezeichnet werden. Ereignisse können beispielsweise eine Änderung des Status der ausgeführten Workflow-Instanz oder das Erzeugen von Daten durch die Workflow-Instanz sein. Vorteilhaft ist es, wenn die beim Ausführen der Workflow-Instanz erzeugten Ereignisse in dem verteilten Repository-Netzwerk gespeichert werden. Die in dem verteilten Repository-Netzwerk gespeicherten Ereignisse können dann als unveränderlicher Audit-Trail für die jeweilige Workflow-Instanz verwendet werden.
- Aufgaben, die einem Benutzer, einer Benutzerrolle oder einem Clientsystem zugeordnet werden, wenn ein Workflow-Schritt in dem Computersystem ausgeführt wird. Sobald eine Aufgabe beim Ausführen einer Workflow-Instanz ausgeführt wurde, wird der nächste Workflow-Schritt oder, sofern vorhanden, die nächste Aktion innerhalb des Workflow-Schrittes angestoßen.
   a) Eine Aufgabe kann einen auszuführenden Workflow-Schritt umfassen.
   b) Alternativ kann eine Aufgabe eine Aktion umfassen, die von einem Benutzer außerhalb des Computersystems ausgeführt werden muss und dessen Ausführung in dem Computersystem bestätigt werden muss. Die Bestätigung kann digital signiert und in dem verteilten Repository-Netzwerk gespeichert werden.
   c) In einer noch weiteren Alternative kann eine Aufgabe eine Aktion umfassen, die beispielsweise (ohne Benutzerinteraktion) von einem Clientsystem ausgeführt werden muss. Beispielsweise kann eine Aufgabe darin bestehen, dass das Clientsystem eine Funktion in einem externen Computersystem ausführt. Nach erfolgreicher Ausführung kann das Clientsystem die Ausführung bestätigen, wobei die Bestätigung vorzugsweise digital signiert und in dem verteilten Repository-Netzwerk gespeichert wird.
- Benachrichtigungen, die einem Benutzer, einer Benutzerrolle oder einem Clientsystem zur Verfügung gestellt werden, wenn ein Workflow-Schritt in dem Computersystem erfolgreich ausgeführt wurde oder wenn ein Workflow-Schritt in dem Computersystem ausgeführt werden muss. Beim Ausführen einer Workflow-Instanz werden die Benachrichtigungen im verteilten Repository-Netzwerk gespeichert.

Vorteilhaft ist es, wenn die Benachrichtigungen verschlüsselt in dem verteilten Repository-Netzwerk gespeichert werden, vorteilhafter Weise mit dem öffentlichen Schlüssel der Instanz (Benutzer, Benutzerrolle oder Clientsystem), für die die Benachrichtigungen bestimmt ist. Die hierfür benötigten öffentlichen Schlüssel können ebenfalls in dem verteilten Repository-Netzwerk gespeichert werden.

Die auf den Clientsystemen ausgeführten Workflow-Applikationen können das verteilte Repository-Netzwerk hinsichtlich neuer Benachrichtigungen überwachen. So kann beispielsweise nach einem erfolgreichen Ausführen eines Workflow-Schrittes einer Workflow-Instanz auf einem ersten Clientsystem eine Benachrichtigung für ein zweites Clientsystem erzeugt und in dem verteilten Repository-Netzwerk gespeichert werden. Mit dieser Benachrichtigung kann das zweite Clientsystem beispielsweise darüber informiert werden, dass der nächste Workflow-Schritt nunmehr ausgeführt werden kann. Sobald das zweite Clientsystem diese Benachrichtigung "erkennt", kann es den nächsten Workflow-Schritt ausführen.

Anstelle des Überwachens des verteilten Repository-Netzwerkes hinsichtlich neuere Benachrichtigungen, können die erzeugten Benachrichtigungen von dem verteilten Repository-Netzwerk auch direkt an die entsprechenden Empfänger gesendet werden, beispielsweise als E-Mail. Die Benachrichtigungen können auch in diesem Fall in dem verteilten Repository-Netzwerk gespeichert werden.

Ferner können in dem Workflow-Modell für jeden Workflow-Schritt Informationen zu einer Anzahl von Aktionen definiert und gespeichert werden, die bei der Ausführung des Workflow-Schrittes einer Workflow-Instanz ausgeführt werden können.

In einer weiteren Ausgestaltung der Erfindung kann es vorgesehen sein, sogenannte Rollback-Informationen oder Rollback-Aktionen in dem Workflow-Modell zu hinterlegen, mit denen bei der Ausführung einer Workflow-Instanz bereits ausgeführte Aktionen und/oder Workflow-Schritte rückgängig gemacht werden können.

In einer Ausgestaltung der Erfindung ist es vorgesehen, dass die Software zur Modellierung von Workflows und/oder das verteilte Repository-Netzwerk ein erzeugtes Workflow-Modell validieren, sodass das Erzeugen einer Workflow-Instanz erst dann möglich ist, wenn das Workflow-Modell erfolgreich validiert wurde. Es kann beispielsweise validiert werden, ob alle in dem Workflow-Modell angegebenen Ausführungsbedingungen valide sind.

Ein Workflow-Modell kann in mehreren Versionen in dem verteilten Repository-Netzwerk gespeichert werden. Vorteilhaft ist es hierbei, wenn eine Workflow-Instanz nur zu der aktuellsten Version des Workflow-Modells erzeugt werden kann. Befindet sich eine Workflow-Instanz in Ausführung, die gestartet wurde, bevor eine neue Version des Workflow-Modells in dem verteilten Repository-Netzwerk gespeichert wurde, dann wird diese Workflow-Instanz gemäß dem Workflow-Modell der älteren Version weiter ausgeführt.

In dem Workflow-Modell kann ein vorbestimmter Workflow-Schritt als Initialschritt definiert werden. Der Initialschritt eines Workflows ist jener Schritt, der beim Starten bzw. Ausführen einer Workflow-Instanz als erster Workflow-Schritt ausgeführt wird. Alternativ hierzu ist der erste Workflow-Schritt in dem Workflow-Modell der Initialschritt.

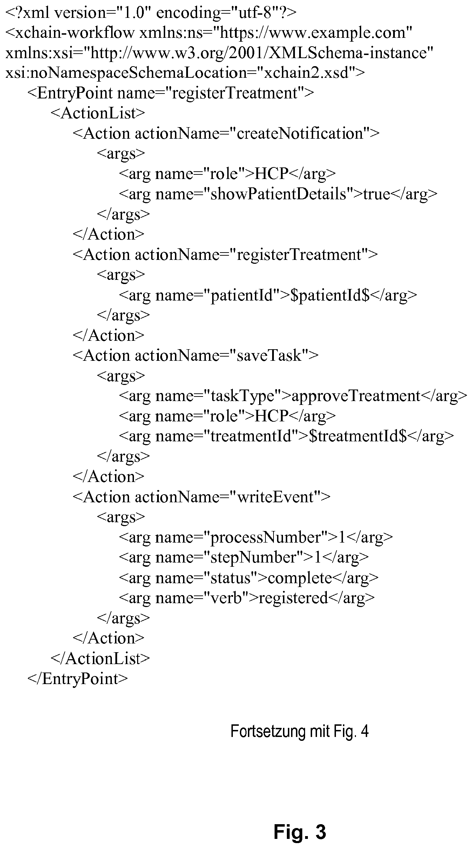

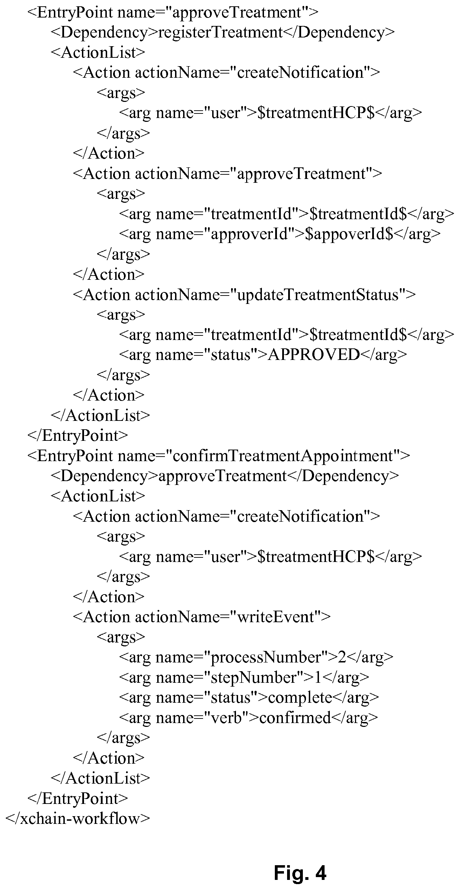

In einer Ausgestaltung der Erfindung kann das Workflow-Modell als XML-Datei erzeugt und in dem verteilten Repository-Netzwerk gespeichert werden. Ein Beispiel eines Workflow-Modells in einer XML-Notation ist in **Fig. 3** und **Fig. 4** gezeigt.

Die Knoten <EntryPoint> repräsentieren hierbei die Workflow-Schritte.

Der Knoten <Dependency> unterhalb des Knotens <EntryPoint> repräsentiert Ausführungsbedingungen, die vor dem Ausführen des jeweiligen Workflow-Schrittes erfüllt sein müssen, damit der Workflow-Schritt ausgeführt werden kann. Beispielsweise gibt der Knoten <Dependency>registerTreatment</Dependency> des Workflow-Schrittes "approveTreatment" (<EntryPoint name="approveTreatment">) an, dass der Workflow-Schritt "registerTreatment" (<EntryPoint name="registerTreatment">) erfolgreich abgeschlossen sein muss, bevor der Workflow-Schritt "approveTreatment" ausgeführt werden kann.

Die Knoten <Action> unterhalb des Knotens <ActionList> repräsentieren die Aktionen, die bei der Ausführung des Workflow-Schrittes einer Workflow-Instanz ausgeführt werden bzw. auszuführen sind. Mit den Konten <arg> können eine Anzahl von Argumenten für die jeweilige Aktion definiert werden. Werte von Argumenten, die zwischen zwei $ gesetzt sind (z.B. $patientId$) repräsentieren hierbei dynamische Werte, die während der Ausführung der Workflow-Instanz gesetzt werden (z.B. von der Workflow-Applikation). Solche dynamischen Werte können entweder in dem verteilten Repository-Netzwerk gespeichert sein und während der Ausführung des jeweiligen Workflow-Schritts aus dem verteilten Repository-Netzwerk ausgelesen und dem Clientsystem bzw. der Workflow-Applikation, die den Workflow-Schritt ausführt zur Verfügung gestellt werden. Alternativ können solche dynamischen Werte auch in der lokalen Speichereinrichtung des den Workflow-Schritt ausführenden Clientsystems gespeichert sein, wobei die jeweilige Workflow-Applikation zur Ausführung des Workflow-Schrittes den entsprechenden Wert aus der lokalen Speichereinrichtung auslesen kann.

Ausgezeichnete Aktionen sind <Action actionName="createNotification">, <Action actionName="saveTask"> und <Action actionName="writeEvent">.

Mit der Aktion "createNotification" wird eine Benachrichtigung erzeugt und in dem verteilten Repository-Netzwerk gespeichert, wobei mit dem Argument "user" der Benutzer bzw. mit dem Argument "role" die Benutzerrolle angegeben wird, für den bzw. die die Benachrichtigung bestimmt ist.

Mit der Aktion "saveTask" werden Aufgaben erzeugt die einem Benutzer oder einer Benutzerrolle zugeordnet werden, wobei mit dem Argument "user" der Benutzer bzw. mit dem Argument "role" die Benutzerrolle angegeben wird, für den bzw. die die Aufgabe bestimmt ist.

Mit der Aktion "writeEvent" werden Ereignisse, die beim Ausführen des Workflow-Schrittes in dem Computersystem auftreten, in dem verteilten Repository-Netzwerk gespeichert. Die Aktion 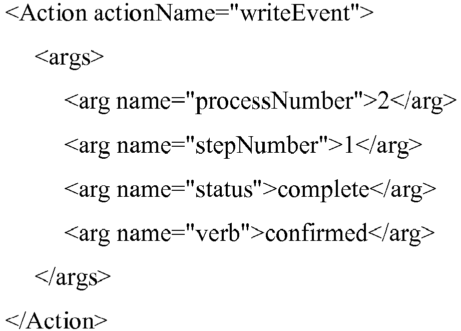 gibt beispielsweise an, dass die durch "<arg name="stepNumber">1</arg>" angegebene Aktion des durch "<arg name="processNumber">2</arg>" angegebenen Workflow-Schrittes abgeschlossen und bestätigt ist. Bei einem als Blockchain ausgestalteten verteilten Repository-Netzwerk kann so der aktuelle Status einer Workflow-Instanz gespeichert werden, während alle vorangegangen gespeicherten Stati unveränderlich erhalten bleiben.

Letztlich kann mit der Aktion "writeEvent" der Status der ausgeführten Workflow-Instanz in dem verteilten Repository-Netzwerk gespeichert werden. Sind mehrere Stati zu einer Workflow-Instanz in dem verteilten Repository-Netzwerk gespeichert, dann gibt der zuletzt gespeicherte Status den aktuellen Status der Workflow-Instanz an.

Das in Fig. 3 und Fig. 4 gezeigte Workflow-Modell beschreibt einen einfachen Workflow für die Behandlung eines Patienten. Er umfasst drei Workflow-Schritte: "registerTreatment", "approveTreatment" und "confirmTreatmentAppointment".

Der Workflow-Schritt "registerTreatment" ist hier der Initialschritt, der beim Erzeugen der Workflow-Instanz ausgeführt wird.

Der Workflow-Schritt "registerTreatment" dient zum Initialisieren der Behandlung und umfasst hier vier Aktionen:
- "createNotification" - hiermit wird bei der Ausführung des Workflow-Schrittes eine Benachrichtigung für die Benutzerrolle "HCP" erstellt, wobei die Benachrichtigung Details zum Patienten enthält. Die Aktion "createNotification" ist die erste Aktion, die beim Ausführen des Initialschrittes ausgeführt wird. Das heißt, beim Erzeugen einer Workflow-Instanz wird als Erstes eine Benachrichtigung für die Benutzerrolle "HCP" erstellt und in dem verteilten Repository-Netzwerk gespeichert.
- "registerTreatment" - hiermit wird ein Behandlungsdatensatz erzeugt und in der lokalen Speichereinrichtung oder in dem verteilten Repository-Netzwerk gespeichert. Der Behandlungsdatensatz wird vorzugsweise verschlüsselt gespeichert. Alternativ kann ein verschlüsselter Hash-Wert des Behandlungsdatensatzes in dem verteilten Repository-Netzwerk gespeichert werden, während der Behandlungsdatensatz selbst in der lokalen Speichereinrichtung des Clientsystems gespeichert wird, in dem der Behandlungsdatensatz erzeugt wird. Ferner wird der Behandlungsdatensatz einem Patientendatensatz zugeordnet, wobei in dem verteilten Repository-Netzwerk lediglich eine eindeutige Kennung des Patientendatensatzes gespeichert und dem Behandlungsdatensatz zugeordnet wird.
- "saveTask" - hiermit eine Aufgabe erzeugt und zur Erledigung einem Benutzer oder einer Benutzerrolle zugeordnet. Bei dem in Fig. 3 gezeigten Beispiel wird die Aufgabe "approveTreatment" erzeugt und der Benutzerrolle "HCP" zugeordnet. Als weiteres Argument wird dieser Aktion eine Kennung der Behandlung bzw. des Behandlungsdatensatzes (treatmentId) übergeben.
- "writeEvent" - hiermit wird ein Ereignis erzeugt, mit dem die Ausführung des Workflow-Schrittes protokolliert wird. Anhand der erzeugten Ereignisse kann die Workflow-Applikation beispielsweise prüfen, ob ein bestimmter Workflow-Schritt korrekt beendet wurde, bevor ein weiterer Workflow-Schritt ausgeführt wird.

Dem Workflow-Schritt "registerTreatment" schließen sich die Workflow-Schritte "approveTreatment" und "confirmTreatmentAppointment" an. Diese beiden Workflow-Schritte weisen jeweils zusätzlich einen Knoten <Dependency> auf, der angibt, von welchem Workflow-Schritt der jeweilige Workflow-Schritt abhängt. Der Workflow-Schritt "approveTreatment" hängt hier beispielsweise von dem Workflow-Schritt "registerTreatment" ab, d.h. der Workflow-Schritt "registerTreatment" muss vollständig und korrekt ausgeführt worden sein, bevor der Workflow-Schritt "approveTreatment" ausgeführt werden kann. Diese Bedingungen können von der jeweiligen Workflow-Applikation geprüft werden.

Die übrigen Knoten der beiden Workflow-Schritte "approveTreatment" und "confirmTreatmentAppointment" entsprechen - entsprechend angepasst - den entsprechenden Knoten des Workflow-Schritts "registerTreatment".

Wie oben beschrieben, wird das auf dem Clientsystem 1 erzeugte Workflow-Modell WM bzw. die entsprechende XML-Datei in dem verteilten Repository-Netzwerk gespeichert. Das Workflow-Modell steht dann, gegebenenfalls nach einer Validierung, zur Ausführung bereit, d.h. es können Workflow-Instanzen des Workflow-Modells erzeugt und ausgeführt werden.

### Hauptschritt 2: Erzeugen einer Workflow-Instanz

Der Hauptschritt 2, d.h. das Erzeugen einer Workflow-Instanz basierend auf dem zuvor erzeugten Workflow-Modell, wird bei dem in Fig. 2 gezeigten Beispiel an dem zweiten Clientsystem (Client 2) ausgeführt. Hierzu ist an dem zweiten Clientsystem eine Workflow-Applikation WA vorgesehen, die auf Anforderung durch den Benutzer des zweiten Clientsystems eine Workflow-Instanz WI des im verteilten Repository-Netzwerk gespeicherten Workflow-Modells WM erzeugt und die erzeugte Workflow-Instanz in dem verteilten Repository-Netzwerk speichert.

Die Workflow-Instanz wird durch Ausführen des in dem Workflow-Modell definierten Initialschrittes erzeugt. Der Initialschritt kann der erste Workflow-Schritt in dem Workflow-Modell sein. Der Initialschritt wird von der Workflow-Applikation ausgeführt.

Zum Ausführen des Initialschrittes müssen die für diesen Workflow-Schritt in dem Workflow-Modell hinterlegen Ausführungsbedingungen erfüllt sein. Beispielsweise muss der Benutzer oder die Benutzerrolle, die den Initialschritt ausführen und damit die Workflow-Instanz erzeugen will, über entsprechende Berechtigungen verfügen. Das Prüfen der entsprechenden Ausführungsbedingungen, insbesondere der Berechtigungen kann von der Workflow-Applikation durchgeführt werden.

Beim Erzeugen der Workflow-Instanz wird ein Abbild des Workflow-Modells erzeugt und in dem verteilten Repository-Netzwerk gespeichert. Das Abbild des Workflow-Modells kann beispielsweise ein JSON-Dokument sein, in dem lediglich der Name und gegebenenfalls die Version des Workflow-Modells, die Namen der Workflow-Schritte, die Namen der Aktionen und die Stati der Workflow-Schritte gespeichert sind. Die Workflow-Instanzen können so deutlich kompakter gespeichert werden als das zugehörige Workflow-Modell. Die kompaktere (d.h. platzsparendere) Speicherung der Workflow-Instanz ist insbesondere deswegen von Bedeutung, da zu einem Workflow-Modell beliebig viele Workflow-Instanzen erzeugt werden können, die alle in dem verteilten Repository-Netzwerk gespeichert werden müssen. Der zuletzt gespeicherte Status einer Workflow-Instanz gibt hierbei jeweils an, wo sich die Workflow-Instanz bei der Ausführung aktuell befindet.

Beim Ausführen bzw. zum Ausführen des Initialschrittes erzeugt die Workflow-Applikation WA des zweiten Clientsystems (Client 2) eine Benachrichtigung N und speichert diese in dem verteilten Repository-Netzwerk. Das Erzeugen der Benachrichtigung N ist die erste auszuführende Aktion in dem Initialschritt. Bei dem in Fig. 3 gezeigten Beispiel ist die Aktion "createNotification" des Workflow-Schrittes "registerTreatment" die erste auszuführende Aktion.

Die Benachrichtigung wird in dem verteilten Repository-Netzwerk der Workflow-Instanz und ggf. einem Workflow-Schritt zugeordnet. Zudem wird in der Benachrichtigung angegeben, für welchen Benutzer bzw. für welche Benutzerrolle die Benachrichtigung erzeugt wurde. In dem genannten Beispiel ist die Benachrichtigung für die Benutzerrolle "HCP" bestimmt.

Für das Erzeugen der Benachrichtigung N verwendet die Workflow-Applikation WA einen Programmcode PC, der in dem verteilten Repository-Netzwerk gespeichert ist und der Aktion "createNotification" zugeordnet ist. Die Workflow-Applikation liest diesen Programmcode aus dem verteilten Repository-Netzwerk aus und bringt ihn auf dem Clientsystem (Client 2) zur Ausführung. Alternativ kann der Programmcode PC auch in der lokalen Speichereinrichtung SE des Clientsystems gespeichert sein und von dort zur Ausführung ausgelesen werden. In einer noch weiteren Alternative kann der PC auch Bestandteil der Workflow-Applikation WA sein.

Anschließend wird der Status der Workflow-Instanz in dem verteilten Repository-Netzwerk gespeichert. In vorliegenden Beispiel umfasst der Status nach dem Ausführen der ersten Aktion Daten darüber, dass sich die Workflow-Instanz nunmehr in dem ersten Workflow-Schritt befindet und dass als nächstes die zweite Aktion innerhalb des ersten Workflow-Schrittes auszuführen ist.

Das Erzeugen der Workflow-Instanz ist damit abgeschlossen.

### Hauptschritt 3: Ausführen der Workflow-Instanz.

Nach dem erfolgreichen Erzeugen der Workflow-Instanz (d.h. nach dem Ausführen des Initialschrittes) können die weiteren in dem zur Workflow-Instanz dazugehörigen Workflow-Modell definierten Workflow-Schritte bzw. die weiteren in dem Initialschritt vorgesehenen Aktionen ausgeführt werden.

Die weiteren Workflow-Schritte bzw. die weiteren in dem Initialschritt vorgesehenen Aktionen können von verschiedenen Clientsystemen bzw. von Workflow-Applikationen verschiedener Clientsysteme ausgeführt werden. Gemäß dem in Fig. 2 gezeigten Beispiel werden dieses weiteren Workflow-Schritte bzw. die weiteren in dem Initialschritt vorgesehenen Aktionen von den Clientsystemen Client 3 bis Client n ausgeführt. Es ist erfindungsgemäß aber auch möglich, dass weitere Workflow-Schritte bzw. weitere Aktionen des Initialschrittes von dem ersten Clientsystem (Client 1), an dem das Workflow-Modell erzeugt wurde, und/oder von dem zweiten Clientsystem (Client 2), an dem die Workflow-Instanz erzeugt wurde, ausgeführt werden. Wo welche Workflow-Schritte konkret ausgeführt werden bzw. zur Ausführung gebracht werden, hängt in erster Linie davon ab, welchen Benutzern bzw. welchen Clientsystemen die jeweiligen Workflow-Schritte zugeordnet sind.

Bei dem in Fig. 3 / Fig. 4 gezeigten Beispiel ist der Workflow-Schritt "approveTreatment" der Benutzerrolle "HCP" zugeordnet, wie in dem Knoten 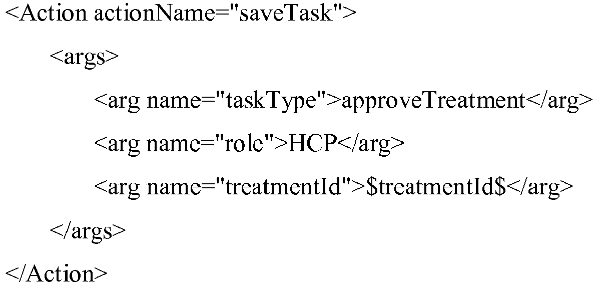 des Workflow-Modells definiert.

Ist nur der Benutzer des dritten Clientsystems (Client 3) der Benutzerrolle "HCP" zugeordnet, dann kann der Workflow-Schritt "approveTreatment" auch nur an dem dritten Clientsystem bzw. von der Workflow-Applikation des dritten Clientsystems ausgeführt werden (denn die Workflow-Applikation des dritten Clientsystems führt die entsprechende Prüfung der Ausführungsbedingungen durch).

Gemäß des in Fig. 3 / Fig. 4 gezeigten Beispiels eines Workflow-Modells ist die nächste nach dem Erzeugen der Workflow-Instanz auszuführende Aktion die Aktion "registerTreatment", wie in dem Knoten 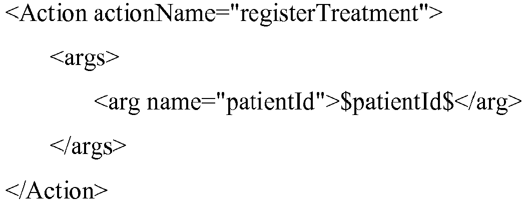 des Workflow-Modells definiert.

Für die nachfolgende Beschreibung wird angenommen, dass die weiteren Workflow-Schritte bzw. die weiteren in dem Initialschritt vorgesehenen Aktionen von bzw. an den Clientsystemen Client 3 und / oder Client n bzw. von den Workflow-Applikationen dieser Clientsysteme ausgeführt werden.

Ferner wird für die nachfolgende Beschreibung angenommen, dass der erste Workflow-Schritt "registerTreatment" (<EntryPoint name ="registerTreatment">) bereits vollständig und erfolgreich ausgeführt wurde.

Mit der Aktion "saveTask" des ersten Workflow-Schritts "registerTreatment" wurde eine Aufgabe erzeugt und zur Erledigung der Benutzerrolle "HCP" zugeordnet. In dem vorliegenden Beispiel besteht die Aufgabe darin, den Workflow-Schritt "approveTreatment" auszuführen.

Mit der Aktion ""writeEvent" des ersten Workflow-Schritts "registerTreatment" wurde der aktuelle Status der Workflow-Instanz in dem verteilten Repository-Netzwerk gespeichert.

Nach Abschluss des ersten Workflow-Schrittes "registerTreatment" wird die erste Aktion (createNotification) des zweiten Workflow-Schrittes ausgeführt. Mit der Aktion "createNotification" wird eine Benachrichtigung N für den in dem Argument "user" angegebenen Benutzer erzeugt. Dem Benutzer wird dadurch mitgeteilt, dass der Workflow-Schritt "approveTreatment" nunmehr ausgeführt werden kann.
- Die erzeugte Benachrichtigung kann von dem Clientsystem, an dem die Benachrichtigung erzeugt wurde, direkt an ein bestimmtes Clientsystem oder an den angegebenen Benutzer übertragen werden, beispielsweise in Form einer E-Mail.
- Alternativ kann die erzeugte Benachrichtigung in dem verteilten Repository-Netzwerk gespeichert werden und das verteilte Repository-Netzwerk kann die Benachrichtigung an ein bestimmtes Clientsystem oder an den angegebenen Benutzer übertragen.
- In einer noch weiteren Alternative kann die Benachrichtigung in dem verteilten Repository-Netzwerk gespeichert werden und die Clientsysteme bzw. die an den Clientsystemen ausgeführten Workflow-Applikationen WA können das verteilte Repository-Netzwerk nach Benachrichtigungen überwachen. Hierzu können die Workflow-Applikationen in regelmäßigen Abständen das verteilte Repository-Netzwerk nach vorhandenen Benachrichtigungen abfragen. Vorteilhaft kann es hierbei sein, wenn ein Workflow-Applikation nur solche Benachrichtigungen abfragt, die für das jeweilige Clientsystem bzw. für den Benutzer des Clientsystems bestimmt sind.

Unabhängig von der Art der Übermittlung der Benachrichtigung, kann es vorteilhaft sein, die Benachrichtigung zu verschlüsseln und im Falle der Speicherung der Benachrichtigung in dem verteilten Repository-Netzwerk die Benachrichtigung verschlüsselt zu speichern.

Nach Empfang einer Benachrichtigung durch das Clientsystem bzw. wenn eine Benachrichtigung für das Clientsystem in dem verteilten Repository-Netzwerk vorliegt, kann das Clientsystem bzw. die jeweilige Workflow-Applikation den entsprechenden Workflow ausführen. Bei dem hier gezeigten Beispiel wird der Benutzer "treatmentHCP" mit der Benachrichtigung darüber informiert, dass der Workflow-Schritt "approveTreatment" nunmehr ausgeführt werden kann.

Der Workflow-Schritt "approveTreatment" kann dann von der Workflow-Applikation des entsprechenden Clientsystems ausgeführt werden. Die erste vom Clientsystem auszuführende Aktion des Workflow-Schritts "approveTreatment" ist hierbei die Aktion "approveTreatment" (<Action name ="approveTreatment">), d.h., die erste Aktion nach der Aktion "CreateNotification".

Um die Aktion "approveTreatment" auszuführen, kann die Workflow-Applikation einen Programmcode zur Ausführung bringen, der der Aktion "approveTreatment" zugeordnet ist. Der entsprechende Programmcode PC kann als Smart Contract oder als Chaincode in dem verteilten Repository-Netzwerk gespeichert sein und zur Ausführung an das entsprechende Clientsystem übertragen werden. Alternativ kann der entsprechende Programmcode auch in der Speichereinrichtung SE des jeweiligen Clientsystems gespeichert sein.

Vor oder beim Ausführen des der Aktion "approveTreatment" zugeordneten Programmcodes kann es vorgesehen sein zu prüfen, ob die Bedingungen für das Ausführen der Aktion erfüllt sind.

Eine der Bedingungen ist in dem Workflow-Modell definiert, nämlich durch das Knoten "<Dependency>registerTreatment</Dependency>". Dieser Knoten gibt an, dass der Workflow-Schritt "approveTreatment" erst dann ausgeführt werden kann, wenn der Workflow-Schritt "registerTreatment" erfolgreich abgeschlossen wurde. Das erfolgreiche Beenden des Workflow-Schritts "registerTreatment" kann die Workflow-Applikation anhand der Stati der Workflow-Instanz ermitteln. Beispielsweise wird als letzte Aktion des Workflow-Schrittes "registerTreatment" die Aktion "writeEvent" ausgeführt, die das erfolgreiche Beenden des Workflow-Schrittes "registerTreatment" als Status in dem verteilten Repository-Netzwerk speichert. Ist dieser Status in dem verteilten Repository-Netzwerk vorhanden, dann kann die Bedingung des Knotens "<Dependency>registerTreatment</Dependency>" von der Workflow-Applikation positiv validiert werden und dieses Ausführungsbedingung ist erfüllt.

Eine weitere Bedingung, die für die Ausführung des Workflow-Schrittes "approveTreatment" erfüllt sein muss, kann die entsprechende Berechtigung des ausführenden Benutzers sein. Gemäß dem in Fig. 3 / Fig. 4 gezeigten Beispiel wurde in dem ersten Workflow-Schritt "registerTreatment" mit der Aktion "saveTask" die Aufgabe "approveTreatment" für die Benutzerrolle "HCP" erzeugt. Das bedeutet, dass der Workflow-Schritt "approveTreatment" nur von einem Benutzer ausgeführt werden kann, dem die Rolle "HCP" zugeordnet ist. Diese Zuordnung kann von der Workflow-Applikation geprüft werden.

In einer Ausgestaltung der Erfindung kann es notwendig sein, dass die Workflow-Applikation für die Ausführung des Programmcodes zusätzliche Daten benötigt. Solche Daten können in der Speichereinrichtung SE des Clientsystems oder in dem verteilten Repository-Netzwerk gespeichert sein. Alternativ oder zusätzlich können solche Daten auch durch den Benutzer bereitgestellt werden. Sofern solche Daten in dem verteilten Repository-Netzwerk gespeichert sind, kann es vorteilhaft sein, wenn dieses Date kryptographisch verschlüsselt oder zumindest signiert sind. Die Verschlüsselung der Daten stellt hierbei eine implizite Bedingung für die Ausführung des jeweiligen Workflow-Schrittes dar - denn wenn die Daten nicht entschlüsselt werden können, kann auch der Workflow-Schritt auch nicht erfolgreich ausgeführt werden. D.h. nur jene Instanzen (Benutzer, Rolle, Workflow-Applikation) können den entsprechenden Workflow-Schritt erfolgreich ausführen, die über einen entsprechenden Schlüssel zum Entschlüsseln der Daten verfügen.

Ferner kann es vorgesehen sein, dass die Workflow-Applikation, die den Programmcode ausführt, Daten erzeugt, die anschließend gespeichert werden müssen. Diese Daten können in der lokalen Speichereinrichtung SE des jeweiligen Clientsystems oder in dem verteilten Repository-Netzwerk gespeichert werden. Für den Fall, dass die Daten in dem verteilten Repository-Netzwerk gespeichert werden, kann es vorteilhaft sein, wenn dieses Daten verschlüsselt gespeichert werden, beispielsweise mit einem öffentlichen Schlüssel jener Instanz (Benutzer, Rolle, Workflow-Applikation), die die Daten anschließend lesen können muss.

Alternativ können anstelle der Daten selbst auch Hash-Werte der Daten in dem verteilten Repository-Netzwerk gespeichert werden, beispielsweise dann, wenn die Daten selbst nicht benötigt werden. So reicht es etwa aus, wenn ein erster Workflow-Schritt zu Patientendaten einen Hashwert berechnet und diesen in dem verteilten Repository-Netzwerk speichert, damit ein zweiter Workflow-Schritt Behandlungsdaten in dem verteilten Repository-Netzwerk den Patientendaten zuordnen kann. Die Behandlungsdaten werden dann in dem verteilten Repository-Netzwerk dem Hashwert zugeordnet. Die Patientendaten selbst können damit ausschließlich in einem bestimmten Clientsystem gespeichert werden und bleiben so geschützt.

Für den Zugriff der Workflow-Applikation auf die in dem Clientsystem gespeicherten Daten bzw. zum Speichern von Daten in dem Clientsystem kann eine sogenannte Trusted Computing Appliance TCA bereitgestellt werden. Eine Trusted Computing Appliance TCA ist eine vertrauenswürdige und speziell gesicherte Umgebung des Clientsystems, in der eine oder mehrere vertrauenswürdige Softwarekomponenten ausgeführt werden. Der Austausch der Daten zwischen der Workflow-Applikation und der Speichereinrichtung des Clientsystems kann über die Trusted Computing Appliance TCA bzw. über die vertrauenswürdigen Softwarekomponenten der Trusted Computing Appliance TCA abgewickelt werden.

Der Quellcode der vertrauenswürdigen Softwarekomponente, nachfolgend auch als Trustlet bezeichnet, wird vorzugsweise von allen Beteiligten des Systems verifiziert.

Zum Abfragen von Daten, die in dem Clientsystem gespeichert sind, durch die Workflow-Applikation kann das Trustlet eine entsprechende Anfrage der Workflow-Applikation entgegennehmen. Das Trustlet führt dann die Abfrage aus und übergibt die abgefragten Daten an die Workflow-Applikation.

Hierbei kann es vorteilhaft sein, wenn das Trustlet die ausgelesenen Daten mit einem dem Trustlet zugeordneten privaten Schlüssel signiert und die signierten Daten der Workflow-Applikation zur Verfügung stellt. Die Workflow-Applikation kann dann die Signatur der Daten mit dem öffentlichen Schlüssel des Trustlets validieren und die Daten nur bei einer erfolgreichen Validierung weiterverarbeiten.

Sollten die an die Workflow-Applikation übergebenen signierten Daten in dem verteilten Repository-Netzwerk gespeichert werden, etwa weil diese in einem nachfolgenden Workflow-Schritt benötigt werden, ist es vorteilhaft, wenn die Daten in signierter Form in dem verteilten Repository-Netzwerk gespeichert werden. Der öffentliche Schlüssel des Trustlets wird ebenfalls in dem verteilten Repository-Netzwerk gespeichert. Damit können auch Workflow-Applikationen, die auf einem anderen Clientsystem ausgeführt werden, die Signatur der Daten mit Hilfe des öffentlichen Schlüssels des signierenden Trustlets validieren.

In einer Ausgestaltung der Erfindung kann jedem Trustlet dasselbe kryptographische Schlüsselpaar zugeordnet werden, sodass nur ein öffentlicher Schlüssel in dem verteilten Repository-Netzwerk gespeichert werden muss. In einer weiteren Ausgestaltung der Erfindung ist es aber möglich, dass jedem Trustlet oder zumindest einigen Trustlets verschiedene kryptographische Schlüsselpaare zugeordnet werden, wobei die jeweiligen öffentlichen Schlüssel in dem verteilten Repository-Netzwerk gespeichert werden.

In einer weiteren Ausgestaltung der Erfindung kann die Trusted Computing Appliance TCA eine Speichereinrichtung aufweisen (eine sogenannten private Speichereinrichtung der TCA), die damit ebenfalls in einer vertrauenswürdigen und speziell gesicherten Umgebung des Clientsystems vorhanden ist. Vorteilhaft ist es hierbei, wenn auf diese private Speichereinrichtung der TCA nur das Trustlet zugreifen kann. Bei dieser Ausgestaltung der Erfindung kann das Trustlet neben Daten aus der Speichereinrichtung SE des Clientsystems auch Daten aus der private Speichereinrichtung der TCA auslesen. Vorteilhaft ist es hierbei, wenn die aus der privaten Speichereinrichtung der TCA ausgelesenen Daten nicht an die Workflow-Applikation übertragen werden. Für die aus der privaten Speichereinrichtung der TCA ausgelesenen Daten kann eine Prüfung durchgeführt werden, ob diese vorbestimmte Bedingungen erfüllen. Das Ergebnis dieser Prüfung kann an die Workflow-Applikation übertragen werden ohne die Daten selbst an die Workflow-Applikation übertragen zu müssen. Das Ergebnis dieser Prüfung kann vor dem Übertragen an die Workflow-Applikation digital signiert werden.

Umgekehrt kann das Trustlet auch Daten, die das Trustlet von der Workflow-Applikation empfängt oder von der privaten Speichereinrichtung der TCA ausliest, in der Speichereinrichtung SE des Clientsystems speichern oder an das Datenverarbeitungssystem DV des Clientsystems übergeben. Auch hierbei können die Daten von dem Trustlet digital signiert werden und vor dem Speichern in der Speichereinrichtung SE des Clientsystems kann die Signatur der Daten validiert werden, beispielsweise von dem Datenverarbeitungssystem DV des Clientsystems.

In einer weiteren Ausgestaltung der Erfindung kann die Workflow-Applikation selbst ein Trustlet sein, das in der Trusted Computing Appliance TCA ausgeführt wird (siehe Client n in Fig. 2). In diesem Fall kann der Programmcode, der für die Ausführung der Workflow-Schritte benötigt wird, Bestandteil des Trustlets sein. Alternativ oder zusätzlich kann der Programmcode auch in dem verteilten Repository-Netzwerk gespeichert werden. Der in dem verteilten Repository-Netzwerk gespeicherte Programmcode wird wie das Trustlet selbst von allen Beteiligten des Systems verifiziert und digital signiert in dem verteilten Repository-Netzwerk gespeichert. Die als Trustlet ausgebildete Workflow-Applikation kann dann den für die Ausführung eines Workflow-Schrittes benötigten Programmcode von dem verteilten Repository-Netzwerk lesen und in der Trusted Computing Appliance TCA zur Ausführung bringen. Die Workflow-Applikation kann hierbei prüfen, ob die Signatur des Programmcodes valide ist, bevor der Programmcode ausgeführt wird.

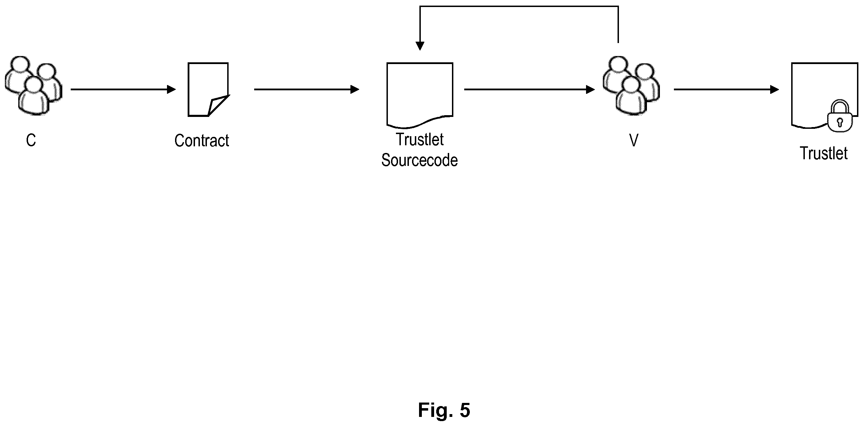

**Fig. 5** zeigt einen exemplarischen Ablauf für das Erzeugen einer vertrauenswürdigen Softwarekomponente (Trustlet).

Eine Gruppe von Inhabern bzw. Besitzern C von vertrauenswürdigen Daten einigen sich auf ein Referenzdokument, etwa ein Vertrag (Contract), in dem die wesentlichen Punkte, sogenannte Checkpoints, für das Validieren von vertrauenswürdigen Daten festgelegt sind.

Basierend auf diesem Referenzdokument bzw. auf die in dem Referenzdokument enthaltenen Checkpoints wird der Quellcode des Trustlets erstellt.

Der Quellcode des Trustlets wird anschließend von einer Person oder mehreren Personen V geprüft bzw. verifiziert. Sollten sich hierbei Fehler, Unstimmigkeiten oder Abweichungen zu den in dem Referenzdokument enthaltenen Checkpoints ergeben, wird der Quellcode des Trustlets angepasst und anschließend erneut geprüft bzw. verifiziert. Dieser Vorgang wird so lange durchgeführt, bis der Source Code des Trustlets keinerlei Abweichungen von den in dem Referenzdokument enthaltenen Checkpoints mehr aufweist und der Source Code des Trustlets damit als verifiziert gilt. Anschließend wird aus dem Source Code des Trustlets das Trustlet erzeugt, beispielsweise in Form eines ausführbaren Programms.

Zu diesem Zeitpunkt kann das Trustlet digital signiert werden. In einer alternativen Ausgestaltung der Erfindung kann das Trustlet dann digital signiert werden, wenn es auf einem Serversystem in einer vertrauenswürdigen und gesicherten Umgebung des Serversystems installiert wird. In dem alternativen Fall kann die digitale Signatur für das Trustlet mit einem dem Serversystem zugeordneten, privaten Schlüssel erzeugt werden. Wenn jedes Serversystem unterschiedliche, private Schlüssel besitzt, können durch Server und Trustlet unterschiedliche digitale Signaturen erzeugt werden, selbst wenn ein und dasselbe Trustlet auf mehreren Serversystemen installiert wird.

Erfindungsgemäß ist es vorgesehen, dass die digitale Signatur in einem verteilten Repository-Netzwerk, etwa in einem Blockchain-Netzwerk, gespeichert wird. Damit kann das auf dem Serversystem installierte Trustlet Daten erzeugen, die erzeugten Daten mit seinem privaten Schlüssel signieren und die signierten Daten beispielsweise an ein Clientsystem übermitteln. Das Clientsystem kann dann mit Hilfe des in dem verteilten Repository-Netzwerk gespeicherten öffentlichen Schlüssels des Serversystems die Signatur der Daten prüfen und die Daten beispielsweise nur dann weiterverarbeiten, wenn die Prüfung der Signatur erfolgreich ist.

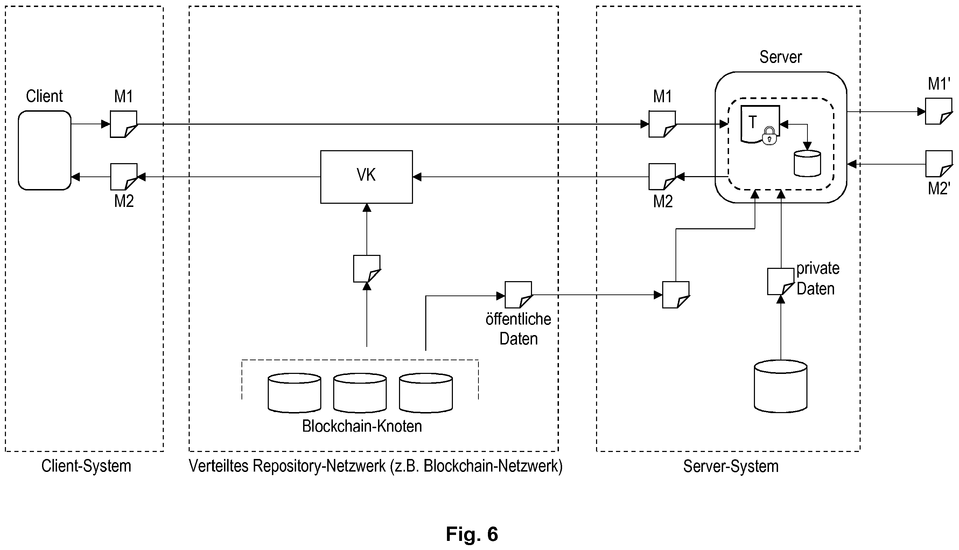

**Fig. 6** zeigt ein Blockdiagramm, anhand dessen das Verfahren zum sicheren Validieren von vertrauenswürdigen Daten näher beschrieben wird.

Fig. 6 zeigt ein Clientsystem, ein mit dem Clientsystem operativ gekoppeltes verteiltes Repository-Netzwerk und ein mit dem verteilten Repository-Netzwerk gekoppeltes Serversystem. Bei dem verteilten Repository-Netzwerk handelt es sich vorzugsweise um ein Blockchain-Netzwerk, das aus mehreren verteilten Blockchain-Knoten gebildet sein kann.

Das Serversystem umfasst im Wesentlichen einen Server, der eine vertrauenswürdige und gesicherte Umgebung (Trusted Execution Environment) bereitstellt. In dieser vertrauenswürdigen und gesicherten Umgebung des Servers wird das Trustlet T installiert. Das Trustlet T wird ausschließlich in dieser vertrauenswürdigen und gesicherten Umgebung ausgeführt.

In einer Ausgestaltung der Erfindung kann in der vertrauenswürdigen und gesicherten Umgebung eine Speichereinrichtung vorhanden sein, die operativ mit dem Trustlet gekoppelt ist. Auf diese Speichereinrichtung kann vorzugsweise nur das Trustlet lesend und schreibend zugreifen. Das Trustlet kann aber auch auf eine außerhalb der vertrauenswürdigen und gesicherten Umgebung vorhandene Speichereinrichtung SE des Serversystems zugreifen, wobei in Fig. 6 nur der lesende Zugriff gezeigt ist.

Beim Installieren des Trustlets in der vertrauenswürdigen und gesicherten Umgebung des Servers wird das Trustlet digital signiert bzw. es wird eine digitale Signatur des Trustlets erzeugt. Die digitale Signatur des Trustlets wird dann in dem verteilten Repository-Netzwerk gespeichert.

Am Client des Clientsystems wird eine erste Validierungs-Anfragenachricht M1 erzeugt, die in dem verteilten Repository-Netzwerk gespeichert wird und an den Server des Serversystems übertragen wird. Die erste Validierungs-Anfragenachricht M1 umfasst hierbei Informationen darüber, welche Daten von dem Server des Serversystems zu validieren sind bzw. welche Daten von dem Server abgefragt werden sollen. Beispielsweise kann mit der ersten Validierungs-Anfragenachricht M1 am Server des Serversystems angefragt werden, ob bestimmte Daten in dem Serversystem gespeichert sind, sei es in der sicheren und vertrauenswürdigen Umgebung vorhandenen Speichereinrichtung, sei es in der Speichereinrichtung SE des Serversystems.

Der Server des Serversystems nimmt die erste Validierungs-Anfragenachricht M1 entgegen und übergibt diese dem in der vertrauenswürdigen und gesicherten Umgebung ausgeführten Trustlet T.

Das Trustlet T liest basierend auf der ersten Validierungs-Anfragenachricht eine Anzahl von Datensätzen aus der Speichereinrichtung SE und/oder aus der in der vertrauenswürdigen und gesicherten Umgebung vorhandenen Speichereinrichtung aus. Nach dem Auslesen der Datensätze erzeugt das Trustlet T eine erste Validierungs-Antwortnachricht M2 und fügt in diese Antwortnachricht die ausgelesenen Datensätze ein.

In einer Ausgestaltung der Erfindung kann es vorgesehen sein, dass das Trustlet für zumindest einen ausgelesenen Datensatz überprüft, ob dieser vorbestimmte Bedingungen erfüllt. Der zumindest eine ausgelesene Datensatz kann auch ein Aggregat von Datensätzen sein bzw. umfassen. In diesem Fall kann das Trustlet in die Validierungs-Antwortnachricht Informationen darüber einfügen, welche der ausgelesenen Datensätze oder das Aggregat der Datensätze die vorbestimmte Bedingung erfüllen, ohne hierbei die Datensätze selbst in die Validierungs-Antwortnachricht M2 einfügen zu müssen.

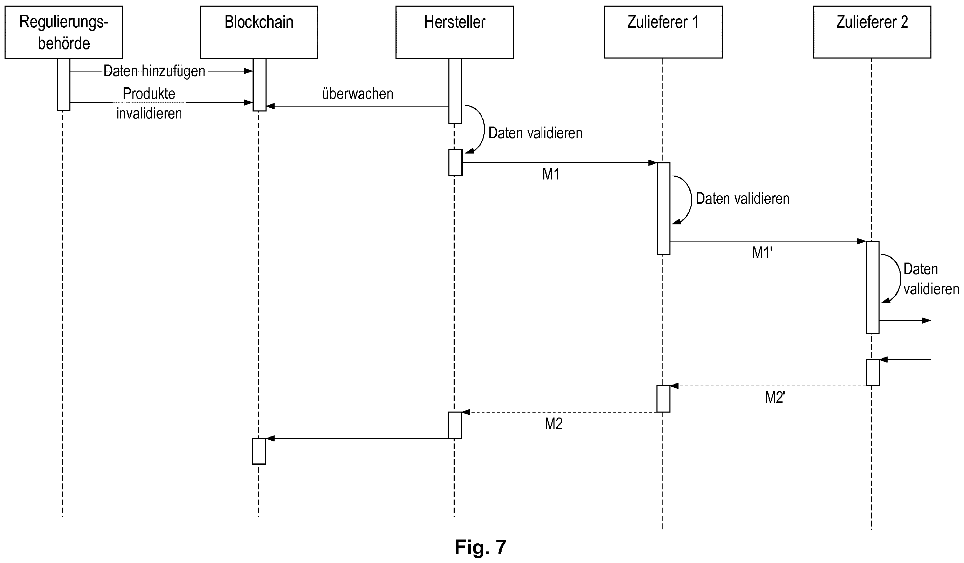

Die Bedingungen, die hierbei geprüft bzw. überprüft werden, können beliebig sein. Ein konkretes Beispiel hierfür wird mit Bezug auf Fig. 7 beschrieben.

In einer Ausgestaltung der Erfindung kann die Validierungs-Anfragenachricht M1 Referenzen zu Daten, die in dem Serversystem (in der Speichereinrichtung SE oder in der vertrauenswürdigen und gesicherten Umgebung vorhandenen Speichereinrichtung) gespeichert sind, und/oder Referenzen zu Daten, die in dem verteilten Repository-Netzwerk gespeichert sind, enthalten. Für die Bearbeitung der Validierungs-Anfragenachricht M1 kann das Trustlet sowohl Daten aus dem verteilten Repository-Netzwerk als auch Daten, die in dem Serversystem gespeichert sind, auslesen.

Die erste Validierungs-Antwortnachricht M2 wird in dem Repository-Netzwerk gespeichert und an das Clientsystem übertragen. Vorteilhaft ist es hierbei, wenn sowohl die erste Validierungs-Anfragenachricht M1, als auch die erste Validierungs-Antwortnachricht M2 verschlüsselt in dem Repository-Netzwerk gespeichert werden.

In einer besonderen Ausgestaltung der Erfindung kann es vorgesehen sein, dass die erste Validierungs-Antwortnachricht M2 von dem Server bzw. von dem Trustlet digital signiert wird, wobei die signierte erste Validierungs-Antwortnachricht in dem verteilten Repository-Netzwerk gespeichert wird. In dem Repository-Netzwerk kann nun die Signatur der ersten Validierungs-Antwortnachricht M2 mit einem öffentlichen Schlüssel des Servers bzw. des Trustlets validiert werden. Der öffentliche Schlüssel des Servers bzw. des Trustlets ist ebenfalls in dem verteilten Repository-Netzwerk gespeichert. Bei einer positiven Validierung kann die Validierungs-Antwortnachricht an das Clientsystem bzw. an den Client übertragen werden. Die Validierung kann von einer Validierungskomponente VK durchgeführt werden, die in dem verteilten Repository-Netzwerk ausgeführt wird.

Diese Art der Validierung der Validierungs-Antwortnachricht M2 kann auch von dem Client selbst durchgeführt werden. Das heißt, die digital signierte erste Validierungs-Antwortnachricht wird in dem verteilten Repository-Netzwerk gespeichert und an den Client übertragen. Der Client validiert dann mit Hilfe des in dem verteilten Repository-Netzwerk gespeicherten öffentlichen Schlüssels des Servers bzw. des Trustlets die Signatur der Validierungs-Antwortnachricht. Bei einer positiven Validierung der Signatur kann das Clientsystem bzw. der Client die erste Validierungs-Antwortnachricht weiterverarbeiten. Anderenfalls kann die erste Validierungs-Antwortnachricht verworfen werden.

Auf dem Serversystem, d.h. in der Speichereinrichtung SE des Serversystems bzw. in der Speichereinrichtung der vertrauenswürdigen und gesicherten Umgebung des Servers können demnach vertrauenswürdige Daten gespeichert werden, während in dem verteilten Repository-Netzwerk nicht-vertrauenswürdige Daten gespeichert werden können. Mit dem erfindungsgemäßen Verfahren ist es damit möglich, auf dem Serversystem gespeicherte vertrauenswürdige Daten zu validieren, ohne dass die Daten selbst offengelegt werden bzw. nur jene Daten offengelegt werden, die ohnehin nicht vertrauenswürdig sind, da sie beispielsweise in dem verteilten Repository-Netzwerk gespeichert sind. Ein entsprechendes Beispiel hierzu wird nachfolgend mit Bezug auf Figur 7 näher beschrieben.

Sofern der Server des Serversystems die Validierungs-Anfragenachricht M1 nicht selbst vollständig beantworten kann, kann es vorgesehen sein, dass das Serversystem basierend auf der ersten Validierungs-Anfragenachricht M1 eine zweite Validierungs-Anfragenachricht M1' erzeugt und diese an ein weiteres Serversystem übergibt und in dem verteilten Repository-Netzwerk speichert. Das weitere Serversystem kann dann in entsprechender Weise eine zweite Validierungs-Antwortnachricht M2' erzeugen und diese in dem verteilten Repository-Netzwerk speichern und an das Serversystem übertragen. In diesem Fall stellt das Serversystem im Bezug auf das weitere Serversystem ein Clientsystem dar. Das weitere Serversystem kann ebenfalls einen Server mit einer gesicherten und vertrauenswürdigen Umgebung aufweisen, in dem ebenfalls ein Trustlet ausgeführt wird. Auf diese Art und Weise kann eine hierarchische bzw. mehrstufige Validierung von vertrauenswürdigen Daten realisiert werden.

**Fig. 7** zeigt ein Ablaufdiagramm für eine mehrstufige bzw. hierarchische Validierung von vertrauenswürdigen Daten.

Der Ablauf wird anhand eines Beispiels beschrieben, bei dem ein Hersteller eines Produktes, beispielsweise eines Kosmetikproduktes, bestimmte Inhaltsstoffe auf der Verpackung bzw. auf dem Etikett angeben muss. Diese deklarierungspflichtigen Inhaltsstoffe werden von einer Regulierungsbehörde vorgegeben. Der Hersteller des Kosmetikproduktes verwendet für die Herstellung ihm bekannte Inhaltsstoffe und kauft bestimmte Komponenten des Kosmetikproduktes von einem ersten Lieferanten zu, wobei der Hersteller die exakte Zusammensetzung dieser Komponenten nicht kennt. Der erste Lieferant kann wiederum Komponenten von einem zweiten Lieferanten zukaufen, wobei der erste Lieferant die Zusammensetzung der von dem zweiten Lieferanten bezogenen Komponenten nicht kennt.

Damit der Hersteller des Kosmetikproduktes alle deklarierungspflichtigen Inhaltsstoffe angeben kann, muss der Hersteller des Kosmetikproduktes sämtliche deklarierungspflichtigen Inhaltsstoffe von sämtlichen Lieferanten in der gesamten Lieferkette abfragen. Eine solche Abfrage dauert im Stand der Technik unter Umständen mehrere Wochen oder gar mehrere Monate, da alle Lieferanten manuell angefragt werden müssen. Im schlimmsten Fall muss so die Auslieferung der hergestellten Kosmetikprodukte gestoppt bzw. verschoben werden. Zudem haben die Zulieferer keinerlei Interesse daran, die Zusammensetzungen der gelieferten Komponenten offenzulegen. Vielmehr wollen die Zulieferer nur die deklarierungspflichtigen Bestandteile der Komponenten offenlegen.

Mit dem erfindungsgemäßen Verfahren zum Validieren von vertrauenswürdigen Daten ist es einerseits möglich, dass nur nicht-vertrauenswürdige Daten (in diesem Beispiel die deklarierungspflichtigen Inhaltsstoffe) offengelegt werden. Andererseits kann der gesamte Validierungsprozess auf wenige Minuten bzw. sogar auf wenige Sekunden reduziert werden, sodass Lieferverzögerungen effektiv verhindert werden.

In dem verteilten Repository-Netzwerk bzw. in der Blockchain werden Daten zu den von der Regulierungsbehörde regulierten Substanzen bzw. Inhaltsstoffen abgelegt. Sämtliche an dem Validierungsprozess beteiligte Instanzen (Hersteller und Zulieferer) können auf diese in der Blockchain gespeicherten Daten zugreifen. Ferner werden von der Regulierungsbehörde solche Produkte registriert, die zumindest einen deklarierungspflichtigen Inhaltsstoff aufweisen.

Sobald Daten eines neuen deklarierungspflichtigen Inhaltsstoffes dem verteilten Repository-Netzwerk hinzugefügt wird bzw. die Daten eines bereits dem verteilten Repository-Netzwerk hinzugefügten Inhaltsstoff von der Regulierungsbehörde geändert werden, werden in dem verteilten Repository-Netzwerk die Daten der Produkte als invalide gekennzeichnet, die einen solchen neuen oder geänderten deklarierungspflichtigen Inhaltsstoff aufweisen.

Der Hersteller des Kosmetikproduktes überwacht die in dem verteilten Repository-Netzwerk gespeicherten Daten. Sobald sich für ihn relevante Daten in dem Repository-Netzwerk geändert haben bzw. neue Daten dem Repository-Netzwerk hinzugefügt wurden, kann der Hersteller des Kosmetikproduktes den Validierungsprozess für seine Produkte starten. Das Überwachen des verteilten Repository-Netzwerks bzw. das Starten des Validierungsprozesses wird von einem dem Hersteller zugeordneten Clientsystem bewerkstelligt.

Sobald das dem Hersteller zugeordnete Clientsystem eine Änderung der in dem verteilten Repository-Netzwerk gespeicherten Daten feststellt, prüft das Clientsystem, welche Komponenten des Kosmetikproduktes von einem externen Zulieferer bereitgestellt werden. Bei dem in Figur 7 gezeigten Ausführungsbeispiel wird eine solche Komponente vom Zulieferer 1 bereitgestellt.

Das Clientsystem erzeugt dann eine erste Validierungs-Anfragenachricht M1 und überträgt diese an das Serversystem des ersten Zulieferers (Zulieferer 1), wobei die erste Validierungs-Anfragenachricht M1 auch in dem verteilten Repository-Netzwerk gespeichert wird. Die erste Validierungs-Anfragenachricht M1 enthält hierbei Informationen dazu, welche Daten von dem Serversystem des ersten Zulieferers zu validieren sind. Im vorliegenden Fall kann die erste Validierungs-Anfragenachricht Informationen darüber umfassen, dass eine bestimmte vom ersten Zulieferer gelieferte Komponente validiert werden muss, d.h., die für diese Komponente regulierten Inhaltsstoffe dem Hersteller bzw. dem Clientsystem mitgeteilt werden sollen.

Der Server des ersten Zulieferers nimmt die erste Validierungs-Anfragenachricht M1 entgegen und übergibt diese dem Trustlet, das in der gesicherten und vertrauenswürdigen Umgebung des Servers ausgeführt wird.

Das Trustlet analysiert nun die Inhaltsstoffe der zu validierenden Komponente, wobei in dem vorliegenden Beispiel ein Inhaltsstoff wiederum eine Komponente ist, die von dem Zulieferer 2 dem Zulieferer 1 geliefert wird.

Für alle Inhaltsstoffe, die nicht von einem weiteren Zulieferer zugeliefert werden, überprüft das Trustlet des Servers des ersten Zulieferers, ob diese vorbestimmte Bedingungen erfüllen. Erfüllt ein Inhaltsstoff die vorbestimmte Bedingung, muss dieser Inhaltsstoff dem Hersteller des Kosmetikproduktes bzw. dem Clientsystem mitgeteilt werden. Eine vorbestimmte Bedingung kann beispielsweise sein, dass es sich bei dem Inhaltsstoff um einen regulierten bzw. deklarationspflichtigen Inhaltsstoff handelt. Zum Überprüfen, ob ein Inhaltsstoff solche Bedingungen erfüllt, führt das Trustlet einen Abgleich der Daten der Inhaltsstoffe mit den im Repository-Netzwerk gespeicherten Daten durch, wobei das Trustlet ermittelt, ob zu den Daten eines Inhaltsstoffes korrespondierende Daten in dem verteilten Repository-Netzwerk gespeichert sind. Die vorbestimmte Bedingung kann hierbei als erfüllt angesehen werden, wenn zu den Daten eines Inhaltsstoffes entsprechende Datensätze in dem Repository-Netzwerk gespeichert sind.

Das Trustlet des Servers des ersten Zulieferers erzeugt nun eine erste Validierungs-Antwortnachricht M2 und fügt dieser Validierungs-Antwortnachricht Informationen zu jenen Inhaltsstoffen hinzu, die die vorbestimmten Bedingungen erfüllen. Inhaltsstoffe, die die vorbestimmten Bedingungen nicht erfüllen, werden der Validierungs-Antwortnachricht M2 nicht hinzugefügt, sodass diese dem Client bzw. dem Hersteller des Kosmetikproduktes nicht offengelegt werden. Die Rezeptur der mit der ersten Validierungs-Anfragenachricht M1 angefragten und zu validierenden Komponente muss damit nicht offengelegt werden und bleibt somit geheim.

Bevor die Validierungs-Antwortnachricht M2 an das Clientsystem übermittelt wird, erzeugt der Server bzw. das Trustlet des Servers des ersten Zulieferers für die Komponenten, die von dem zweiten Zulieferer zugeliefert werden, in entsprechender Weise eine zweite Validierungs-Anfragenachricht M1' und überträgt diese an den Server des zweiten Zulieferers.

Der Server des zweiten Zulieferers nimmt diese zweite Validierungs-Anfragenachricht M1' entgegen und übergibt diese dem Trustlet des Servers, das ebenfalls in einem gesicherten und vertrauenswürdigen Bereich des Servers ausgeführt wird. Das Trustlet des Servers des zweiten Zulieferers überprüft ebenfalls, welche Inhaltsstoffe der angefragten Komponente vorbestimmte Bedingungen erfüllen, beispielsweise deklarierungspflichtig oder reguliert sind. Die Vorgehensweise ist hierbei analog zu dem Trustlet des Servers des ersten Zulieferers. Das heißt, das Trustlet des Servers des zweiten Zulieferers überprüft, ob zu den Inhaltsstoffen der zu validierenden Komponente entsprechende Daten in dem verteilten Repository-Netzwerk gespeichert sind.

Das Trustlet des Servers des zweiten Zulieferers erzeugt nun eine zweite Validierungs-Antwortnachricht M2' und fügt dieser Informationen zu jenen Inhaltsstoffen hinzu, die die vorbestimmten Bedingungen erfüllen. Die Inhaltsstoffe, die die vorbestimmten Bedingungen nicht erfüllen, werden der Validierungs-Antwortnachricht M2' nicht hinzugefügt, das heißt, sie bleiben geheim.

Sofern die von dem Trustlet des zweiten Zulieferers zu validierende Komponente keine Inhaltsstoffe aufweist, die von einem weiteren Zulieferer zugeliefert werden, überträgt das Trustlet die Validierungs-Antwortnachricht M2' an den Server des ersten Zulieferers.

Für den Fall, dass die von dem Trustlet des zweiten Zulieferers zu überprüfende Komponente Komponenten enthält, die von einem weiteren Zulieferer zugeliefert werden, müssen diese Komponenten von einem Trustlet der weiteren Zulieferer validiert werden, wobei hierbei die jeweilige Validierung analog zu dem von den Trustlets des ersten und des zweiten Zulieferers durchgeführten Validierungen durchgeführt wird.

Der Server des ersten Zulieferers empfängt die zweite Validierungs-Antwortnachricht M2' von dem Trustlet des zweiten Zulieferers. Die von dem Server empfangene Validierungs-Antwortnachricht M2' wird an das Trustlet des Servers übergeben. Das Trustlet des Servers des ersten Zulieferers kann nun die mit der zweiten Validierungs-Antwortnachricht M2' empfangenen Daten in die erste Validierungs-Antwortnachricht M2 einfügen.

Anschließend überträgt das Trustlet die erste Validierungs-Antwortnachricht M2 an das Clientsystem des Herstellers des Kosmetikproduktes. Der Hersteller des Kosmetikproduktes hat nun alle Informationen zu regulierten bzw. deklarierungspflichtigen Inhaltsstoffen des Kosmetikproduktes, die in den Komponenten der Zulieferer enthalten sind, sodass der Hersteller Informationen zu diesen Daten entsprechend auf der Verpackung ausweisen kann.

Das Clientsystem des Herstellers muss nun nur noch die Inhaltsstoffe validieren, die nicht in den Komponenten der Zulieferer enthalten sind. Hierzu wird auch auf dem Clientsystem ein Trustlet in einem vertrauenswürdigen und gesicherten Bereich des Clientsystems ausgeführt. Die Validierung dieser Inhaltsstoffe erfolgt analog zu der Validierung, die die Trustlets an den beiden Serversystemen durchführen.

Sämtliche Validierungs-Anfragenachrichten und Validierungs-Antwortnachrichten werden in dem verteilten Repository-Netzwerk gespeichert und gegebenenfalls verschlüsselt und signiert.

Abschließend werden die in dem verteilten Repository-Netzwerk gespeicherten Daten des Produktes von dem Trustlet des Clientsystems als valide gekennzeichnet.

Mit Hilfe der Trustlets, die erfindungsgemäß ausgeführt werden, kann demnach gewährleistet werden, dass vertrauenswürdige Daten auch bei der Ausführung einer Workflows in einem verteilten System vertrauenswürdig bleiben.

## Patentansprüche

1. Computer-implementiertes Verfahren zum sicheren Ausführen eines Workflows in einem Computersystem, das zumindest ein verteiltes Repository-Netzwerk und eine Anzahl von mit diesem Repository-Netzwerk operativ gekoppelte oder operativ koppelbare Clientsysteme umfasst, wobei
- in dem Repository-Netzwerk ein Modell des Workflows gespeichert wird,
- das Modell des Workflows eine Anzahl von Workflow-Schritten umfasst, wobei ein vorbestimmter Workflow-Schritt als Initialschritt, der beim Ausführen des Workflows in dem Computersystem als erster Schritt ausgeführt wird, definiert ist, und wobei für jeden Workflow-Schritt in dem Modell des Workflows gespeichert sind
- Ausführungsbedingungen, die zum Ausführen des Workflow-Schritts in dem Computersystem erfüllt sein müssen,
- Ereignisse, die beim Ausführen des Workflow-Schrittes in dem Computersystem aufgezeichnet werden,
- Aufgaben, die einem Benutzer, einer Benutzerrolle oder einem Clientsystem zugeordnet werden, wenn ein Workflow-Schritt in dem Computersystem ausgeführt wird,
- Benachrichtigungen, die einem Benutzer, einer Benutzerrolle oder einem Clientsystem zur Verfügung gestellt werden, wenn ein Workflow-Schritt in dem Computersystem erfolgreich ausgeführt wurde oder wenn ein Workflow-Schritt in dem Computersystem ausgeführt werden muss, und
- Informationen zu einer Anzahl von Aktionen,
- zum Ausführen des Workflows in dem Computersystem aus dem Modell des Workflows eine Workflow-Instanz erzeugt wird, die den auszuführenden Workflow repräsentiert, wobei die Workflow-Instanz in dem Repository-Netzwerk gespeichert wird,
- beim Ausführen der Workflow-Instanz in dem Computersystem
- zunächst der Initialschritt und anschließend die weiteren Workflow-Schritte ausgeführt werden, wobei beim Ausführen eines Workflow-Schrittes die Aktionen ausgeführt werden,
- beim Starten eines Workflow-Schritts die Ereignisse, die Aufgaben, die Benachrichtigungen und basierend auf den Informationen zu der Anzahl von Aktionen die Aktionen erzeugt und in dem Repository-Netzwerk gespeichert werden,
- für jeden ausgeführten Workflow-Schritt Zustandsdaten in dem Repository-Netzwerk gespeichert werden, und
- eine auf zumindest einem Clientsystem ausgeführte Workflow-Applikation
- Benachrichtigungen von dem Repository-Netzwerk empfängt, die für einen vorbestimmten Benutzer in dem Repository-Netzwerk gespeichert wurden, und
- für einen auszuführenden Workflow-Schritt auf dem zumindest einen Clientsystem einen dem Workflow-Schritt zugeordneten Programmcode ausführt.

2. Verfahren nach Anspruch 1, wobei der einem Workflow-Schritt zugeordnete Programmcode in dem Repository-Netzwerk oder als Bestandteil der Workflow-Applikation auf dem Clientsystem gespeichert wird.

3. Verfahren nach dem Anspruch 1, wobei die Zustandsdaten einen Zeitstempel, der den Ausführungszeitpunkt des Workflow-Schrittes angibt, eine Benutzerkennung des Benutzers, der den Workflow-Schritt ausgeführt hat, und einen Status des Workflow-Schrittes umfassen.

4. Verfahren nach Anspruch1, wobei das verteilte Repository-Netzwerk ein Blockchain-Netzwerk ist.

5. Verfahren nach dem Anspruch 1, wobei beim Ausführen des dem Workflow-Schritt zugeordneten Programmcodes durch die Workflow-Applikation der Workflow-Applikation Daten übergeben werden, die zur Ausführung des Programmcodes notwendig sind.

6. Verfahren nach dem vorhergehenden Anspruch, wobei die übergebenen Daten in einer Speichereinrichtung des Clientsystems, auf dem der Programmcode ausgeführt wird, und/oder in dem verteilten Repository-Netzwerk gespeichert sind.

7. Verfahren nach dem Anspruch 1, wobei beim Ausführen des dem Workflow-Schritt zugeordneten Programmcodes durch die Workflow-Applikation Daten erzeugt werden, wobei die erzeugten Daten in einer Speichereinrichtung des Clientsystems, auf dem der Programmcode ausgeführt wird, und/oder in dem verteilten Repository-Netzwerk gespeichert werden.

8. Verfahren nach dem vorhergehenden Anspruch, wobei die erzeugten Daten verschlüsselt in dem Repository-Netzwerk gespeichert werden.

9. Verfahren nach dem Anspruch 1, wobei das Modell des Workflows einen ersten und zumindest einen zweiten Teilworkflow umfasst, wobei jeder Teilworkflow eine Anzahl von Workflow-Schritte umfasst, und wobei ein Modell des ersten Teilworkflows in einem ersten Clientsystem der Anzahl von Clientsystemen und ein Modell des zumindest einen zweiten Teilworkflows in einem zweiten Clientsystem der Anzahl von Clientsystemen erzeugt wird, wobei die Modelle der Teilworkflows kombiniert werden und als Modell des Workflows in dem verteilten Repository-Netzwerk gespeichert wird.

10. Verfahren nach dem vorhergehenden Anspruch, wobei jedem Modell eines Teilworkflows ein Benutzer und/oder eine Benutzerrolle zugeordnet werden, wobei mit dieser Zuordnung dem Benutzer und/oder der Benutzerrolle Rechte zum Verwalten, insbesondere Rechte zum Ändern des jeweiligen Modells des Teilworkflows, eingeräumt werden.

11. Verfahren nach Anspruch 1, wobei vor dem Ausführen eines Workflow-Schritts von der Workflow-Applikation geprüft wird, ob die Ausführungsbedingungen zum Ausführen des Workflow-Schritts erfüllt sind.

12. Verfahren nach dem vorhergehenden Anspruch, wobei die Ausführungsbedingungen zumindest eines aus der Gruppe
- der auszuführende Workflow-Schritt muss ein gültiger Workflow-Schritt sein, wobei ein auszuführender Workflow-Schritt dann gültig ist, wenn vorangegangene Workflow-Schritte, von denen der auszuführende Workflow-Schritt abhängt, erfolgreich ausgeführt wurden,
- der Benutzer, der den auszuführenden Workflow-Schritt startet, muss eine Berechtigung zum Ausführen des Workflow-Schritts haben, wobei die Berechtigungen in dem verteilten Repository-Netzwerk gespeichert werden, und
- Kombination hiervon,
umfasst.

## Claims

1. Computer-implemented method for securely executing a workflow in a computer system that comprises at least one distributed repository network and a number of client systems which are or can be operatively coupled to this repository network, wherein
- a model of the workflow is stored in the repository network,
- the model of the workflow comprises a number of workflow steps, wherein a predetermined workflow step is defined as an initial step which is executed as a first step when executing the workflow in the computer system, and wherein for each workflow step in the model of the workflow the following are stored
- execution conditions that must be met for executing the workflow step in the computer system,
- events which are recorded when executing the workflow step in the computer system,
- tasks assigned to a user, a user role or a client system when a workflow step is executed in the computer system,
- notifications provided to a user, a user role or a client system when a workflow step has been successfully executed in the computer system or when a workflow step needs to be executed in the computer system, and
- information about a number of actions,
- for executing the workflow in the computer system, a workflow instance which represents the workflow to be executed is generated from the model of the workflow, wherein the workflow instance is stored in the repository network,
- when executing the workflow instance in the computer system
- initially the initial step and subsequently the further workflow steps are executed, wherein the actions are executed during execution of a workflow step,
- when starting a workflow step, the events, the tasks, the notifications and, based on the information about the number of actions, the actions are generated and stored in the repository network,
- state data are stored in the repository network for each executed workflow step, and
- a workflow application executed on at least one client system
- receives notifications from the repository network which were stored in the repository network for a predetermined user, and
- executes, for a workflow step to be executed on the at least one client system, a program code assigned to the workflow step.

2. Method according to claim 1, wherein the program code assigned to a workflow step is stored in the repository network or as a component of the workflow application on the client system.

3. Method according to claim 1, wherein the state data comprise a time stamp indicating the execution time of the workflow step, a user identifier of the user who has executed the workflow step, and a status of the workflow step.

4. Method according to claim 1, wherein the distributed repository network is a blockchain network.

5. Method according to claim 1, wherein when the workflow application executes the program code assigned to the workflow step, data which are necessary for executing the program code are transferred to the workflow application.

6. Method according to the preceding claim, wherein the transferred data are stored in a memory device of the client system on which the program code is executed and/or in the distributed repository network.

7. Method according to claim 1, wherein data are generated when the workflow application executes the program code assigned to the workflow step, wherein the generated data are stored in a memory device of the client system on which the program code is executed and/or in the distributed repository network.

8. Method according to the preceding claim, wherein the generated data are stored in encrypted form in the repository network.

9. Method according to claim 1, wherein the model of the workflow comprises a first and at least one second sub-workflow, each sub-workflow comprising a number of workflow steps, and wherein a model of the first sub-workflow is generated in a first client system of the number of client systems and a model of the at least one second sub-workflow is generated in a second client system of the number of client systems, wherein the models of the sub-workflows are combined and stored as a model of the workflow in the distributed repository network.

10. Method according to the preceding claim, wherein each model of a sub-workflow is assigned a user and/or a user role, wherein with this assignment the user and/or the user role are granted management rights, in particular rights to change the relevant model of the sub-workflow.

11. Method according to claim 1, wherein prior to executing a workflow step, the workflow application checks whether the execution conditions for executing the workflow step are met.

12. Method according to the preceding claim, wherein the execution conditions comprise at least one of the group of
- the workflow step to be executed must be a valid workflow step, wherein a workflow step to be executed is valid when previous workflow steps on which the workflow step to be executed is dependent were successfully executed,
- the user who starts the workflow step to be executed must have authorization to execute the workflow step, wherein the authorizations are stored in the distributed repository network, and
- a combination thereof.

## Revendications

1. Procédé mis en oeuvre par ordinateur pour l'exécution sécurisée d'un flux de travail dans un système informatique qui comprend au moins un réseau de dépôt distribué et un certain nombre de systèmes clients couplés de manière opérationnelle ou pouvant être couplés de manière opérationnelle à ce réseau de dépôt, dans lequel
- un modèle du flux de travail est mémorisé dans le réseau de dépôt,
- le modèle du flux de travail comprend un certain nombre d'étapes de flux de travail, dans lequel une étape de flux de travail prédéterminée est définie comme étape initiale, laquelle est exécutée comme première étape lors de l'exécution du flux de travail dans le système informatique, et dans lequel, pour chaque étape de flux de travail dans le modèle du flux de travail, sont mémorisés
- des conditions d'exécution qui doivent être satisfaites pour l'exécution de l'étape de flux de travail dans le système informatique,
- des événements qui sont enregistrés dans le système informatique lors de l'exécution de l'étape de flux de travail,
- des tâches qui sont attribuées à un utilisateur, à un rôle d'utilisateur ou à un système client lorsqu'une étape de flux de travail est exécutée dans le système informatique,
- des notifications qui sont mises à la disposition d'un utilisateur, d'un rôle d'utilisateur ou d'un système client lorsqu'une étape de flux de travail a été exécutée avec succès dans le système informatique ou lorsqu'une étape de flux de travail doit être exécutée dans le système informatique, et
- des informations concernant un certain nombre d'actions,
- pour l'exécution du flux de travail dans le système informatique, une instance de flux de travail est générée à partir du modèle du flux de travail, laquelle instance de flux de travail représente le flux de travail à exécuter, dans lequel l'instance de flux de travail est mémorisée dans le réseau de dépôt,
- lors de l'exécution de l'instance de flux de travail dans le système informatique
- d'abord l'étape initiale et ensuite les autres étapes de flux de travail sont exécutées, dans lequel les actions sont exécutées lors de l'exécution d'une étape de flux de travail,
- lors du démarrage d'une étape de flux de travail, les événements, les tâches, les notifications et, sur la base des informations concernant le nombre d'actions, les actions sont générées et mémorisées dans le réseau de dépôt,
- pour chaque étape de flux de travail exécutée, des données d'état sont mémorisées dans le réseau de dépôt, et
- une application de flux de travail exécutée sur au moins un système client
- reçoit des notifications du réseau de dépôt, lesquelles ont été mémorisées pour un utilisateur prédéterminé dans le réseau de dépôt, et
- exécute un code de programme attribué à l'étape de flux de travail, pour une étape de flux de travail à exécuter sur l'au moins un système client.

2. Procédé selon la revendication 1, dans lequel le code de programme attribué à une étape de flux de travail est mémorisé dans le réseau de dépôt ou en tant que partie de l'application de flux de travail sur le système client.

3. Procédé selon la revendication 1, dans lequel les données d'état comprennent un horodatage qui indique le moment d'exécution de l'étape de flux de travail, un identifiant d'utilisateur de l'utilisateur qui a exécuté l'étape de flux de travail, et un statut de l'étape de flux de travail.

4. Procédé selon la revendication 1, dans lequel le réseau de dépôt distribué est un réseau à chaîne de blocs.

5. Procédé selon la revendication 1, dans lequel, lors de l'exécution du code de programme attribué à l'étape de flux de travail par l'application de flux de travail, des données sont transmises à l'application de flux de travail, lesquelles données sont nécessaires pour l'exécution du code de programme.

6. Procédé selon la revendication précédente, dans lequel les données transmises sont mémorisées dans un dispositif de mémoire du système client sur lequel le code de programme est exécuté, et/ou dans le réseau de dépôt distribué.

7. Procédé selon la revendication 1, dans lequel, lors de l'exécution du code de programme attribué à l'étape de flux de travail par l'application de flux de travail, des données sont générées, dans lequel les données générées sont mémorisées dans un dispositif de mémoire du système client sur lequel le code de programme est exécuté, et/ou dans le réseau de dépôt distribué.

8. Procédé selon la revendication précédente, dans lequel les données générées sont mémorisées de manière chiffrée dans le réseau de dépôt.

9. Procédé selon la revendication 1, dans lequel le modèle du flux de travail comprend un premier et au moins un second flux de travail partiel, dans lequel chaque flux de travail partiel comprend un certain nombre d'étapes de flux de travail, et dans lequel un modèle du premier flux de travail partiel est généré dans un premier système client du nombre de systèmes clients et un modèle de l'au moins un second flux de travail partiel est généré dans un second système client du nombre de systèmes clients, dans lequel les modèles des flux de travail partiels sont combinés et mémorisés en tant que modèle du flux de travail dans le réseau de dépôt distribué.

10. Procédé selon la revendication précédente, dans lequel un utilisateur et/ou un rôle d'utilisateur est attribué à chaque modèle d'un flux de travail partiel, dans lequel des droits de gestion, en particulier des droits de modification du modèle respectif du flux de travail partiel, sont accordés à l'utilisateur et/ou au rôle d'utilisateur par le biais de cette attribution.

11. Procédé selon la revendication 1, dans lequel, avant l'exécution d'une étape de flux de travail par l'application de flux de travail, il est vérifié que les conditions d'exécution pour l'exécution de l'étape de flux de travail sont satisfaites.

12. Procédé selon la revendication précédente, dans lequel les conditions d'exécution comprend au moins l'un des éléments suivants
- l'étape de flux de travail à exécuter doit être une étape de flux de travail valable, dans lequel une étape de flux de travail à exécuter est valable uniquement lorsque des étapes de flux de travail précédentes, dont l'étape de flux de travail à exécuter dépend, ont été exécutées avec succès,
- l'utilisateur qui démarre l'étape de flux de travail à exécuter doit avoir une autorisation d'exécution de l'étape de flux de travail, dans lequel les autorisations sont mémorisées dans le réseau de dépôt distribué, et
- une combinaison de ceux-ci.
